# EUROPEAN PATENT APPLICATION

(11) **EP 4 763 227 A1**
(43) Date of publication of application: **24.06.2026**
(21) Application number: 24221582.0
(22) Date of filing: 19.12.2024
(51) Int. Cl.: A61K 47/61, A61P 3/00, C07K 14/435, A61K 47/69

(54) **FUNCTIONALIZED BRANCHED-CHAIN AMINO ACID-DEGRADING ENZYME COMPOSITIONS**

(71) Applicant: Perseo Pharma AG, 4132 Muttenz (CH)
(72) Inventor: LAPRÉVOTTE, Emilie, 68220 Hégenheim (FR); BRIAND, Manon, 5408 Ennetbaden (CH); SPECIOSO, Gabriele, 4303 Kaiseraugst (CH)
(74) Representative: Latscha Schöllhorn Partner AG

(57) **Abstract**

The present invention relates to a composition comprising a solid carrier, a protein branched-chain amino acid-degrading enzyme or a fragment thereof immobilized on the surface of the solid carrier, a protective layer to protect the protein branched-chain amino acid-degrading enzyme or a fragment thereof by embedding the protein branched-chain amino acid-degrading enzyme or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group. The present invention also relates to methods of producing said composition and uses of the composition for the prevention, delay of progression or treatment of branched-chain amino acid metabolism related diseases.

## Description

### The field of the invention

The present invention relates to a composition comprising a solid carrier, a branched-chain amino acid-degrading enzyme or a fragment thereof immobilized on the surface of the solid carrier, a protective layer to protect the branched-chain amino acid-degrading enzyme or a fragment thereof by embedding the branched-chain amino acid-degrading enzyme or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group. The present invention also relates to methods of producing said composition and uses of the composition for the prevention, delay of progression or treatment of branched-chain amino acid metabolism related diseases.

### Background of the invention

Branched-chain amino acid metabolism related diseases like Maple Syrup Urine Disease (MSUD) are rare metabolic disorders resulting normally from deficiencies in the branched-chain α-ketoacid dehydrogenase (BCKDH) complex, leading to a toxic accumulation of branched-chain amino acids. Current treatments, such as dietary restrictions or liver transplants, are limited by patient compliance, dietary constraints, and potential complications. Thus, there is a need to provide effective treatments for branched-chain amino acid metabolism related diseases.

### Summary of the invention

The present invention provides a composition comprising a solid carrier, a branched-chain amino acid-degrading enzyme or a fragment thereof immobilized on the surface of the solid carrier, a protective layer to protect the branched-chain amino acid-degrading enzyme or a fragment thereof by embedding the branched-chain amino acid-degrading enzyme or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group.

The present invention provides also a method of producing said composition comprising a solid carrier, a branched-chain amino acid-degrading enzyme or a fragment thereof immobilized on the surface of the solid carrier, a protective layer to protect the branched-chain amino acid-degrading enzyme or a fragment thereof by embedding the branched-chain amino acid-degrading enzyme or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group, the method comprising the following steps:
(a) providing a solid carrier;
(b) immobilizing a branched-chain amino acid-degrading enzyme or a fragment thereof on the solid carrier;
(c) forming a protective layer on the surface of the solid carrier to protect the branched-chain amino acid-degrading enzyme or a fragment thereof immobilized on the solid carrier;
(d) immobilizing a functional constituent on the surface of the protective layer, wherein the functional constituent immobilized on the surface of the protective layer is a polymer comprising repeat units wherein each repeat unit comprises at least one amino group and/or at least one thiol group.

The present invention also provides a method for the prevention, delay of progression or treatment of branched-chain amino acid metabolism related diseases using the composition of the present invention.

It has been surprisingly found by the inventors of the present application that compositions as provided by the present invention if applied therapeutically demonstrate both safety and efficacy in metabolizing leucine and facilitating the absorption of its metabolic product, isopentylamine.

### Brief description of the figures

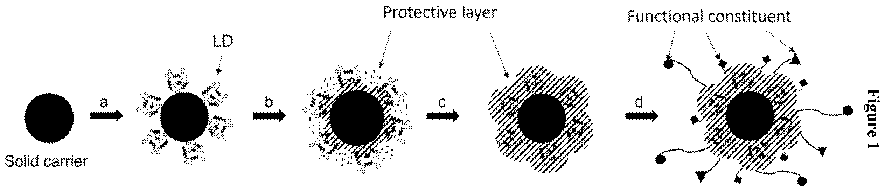

**Figure 1****)** shows a schematic representation of the process for the production of the composition of the invention: a) Leucine decarboxylase (LD) or fragment thereof is immobilized on the solid carrier; b) and c) a protective layer grows around the immobilized LD or fragment thereof embedding the immobilized LD or fragment thereof; and d) a functional constituent is immobilized on the surface of the protective layer.

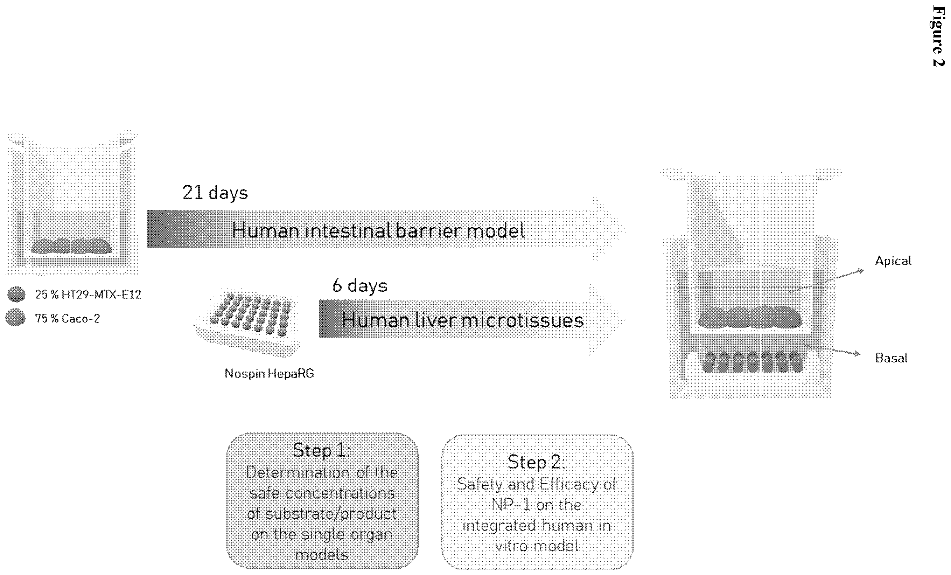

**Figure 2****):** Schematic representation with experimental design and timeline of the single and integrated human *in vitro* models
**Figure 3****):** Safety of different concentrations of leucine in GI inserts. A) Cell viability assessed by the intracellular ATP content. B) Integrity of the barrier registered through the TEER measurement. Percentage of control calculated using the untreated GI inserts as a reference
**Figure 4****):** Safety of different concentrations of isopentylamine in GI inserts. A) Cell viability assessed by the intracellular ATP content. B) Integrity of the barrier registered through the TEER measurement. Percentage of control calculated using the untreated GI inserts as a reference
**Figure 5****):** Safety of different concentrations of leucine in LMTs. A) Cell viability assessed by the intracellular ATP content. B) Release of human Albumin in the extracellular compartment registered through Albumin Elisa. Percentage of control calculated using the untreated LMTs as a reference.
**Figure 6****):** Safety of different concentrations of isopentylamine in LMTs. A) Cell viability assessed by the intracellular ATP content. B) Release of human Albumin in the extracellular compartment registered through Albumin Elisa. Percentage of control calculated using the untreated LMTs as a reference.
**Figure 7****):** Scanning Electron Microscopy of Leucine dexarboxylase-based SNPs (NP-1). A) SEM micrograph of NP-1. B) Size distribution of NP-1 diameter.

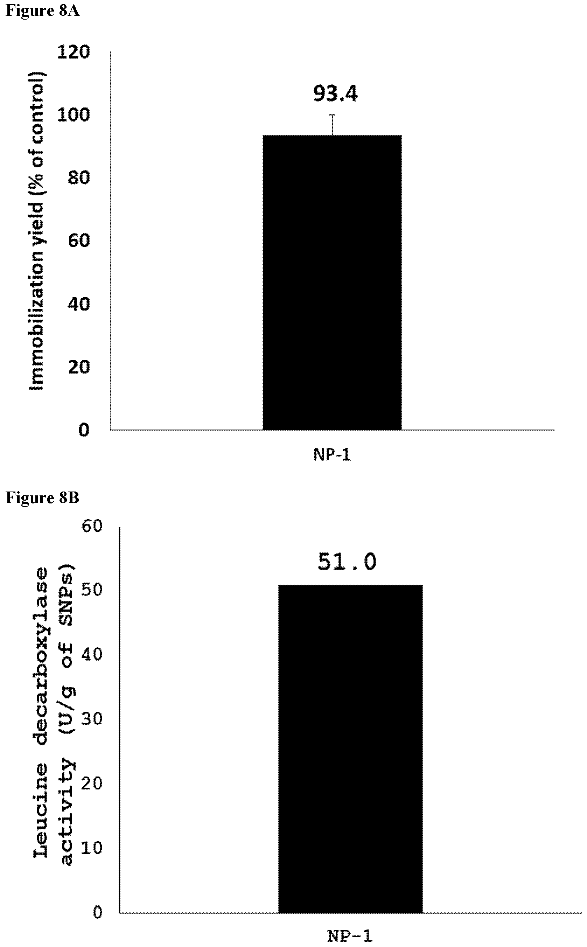

**Figure 8****):** Characterization of NP-1. A) Leucine decarboxylase immobilization efficiency: amount of enzyme immobilized in NP-1 compared with the total amount used for the immobilization process. B) Leucine decarboxylase specific activity per gram of NP-1.
**Figure 9****):** Safety of NP-1 treatment on single or integrated *in vitro* models. A) Safety of different treatment conditions on the GI single model or integrated with LMTs using the intracellular ATP content assay. B) Safety of different treatment conditions on the LMTs single model or integrated with GI inserts using intracellular ATP content assay. Percentage of control calculated using the untreated single or integrated GI inserts or LMTs as a reference.
**Figure 10****):** Efficacy of NP-1. Number of mol (nmol) of isopentylamine produced by NP-1 from leucine on the apical side and subsequentially diffused through the GI model on the basal compartment, in contact with LMTs.

### Detailed description of the invention

The present invention relates to a composition comprising a solid carrier, a branched-chain amino acid-degrading enzyme or a fragment thereof immobilized on the surface of the solid carrier, a protective layer to protect the branched-chain amino acid-degrading enzyme or a fragment thereof by embedding the branched-chain amino acid-degrading enzyme or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group.

For the purposes of interpreting this specification, the following definitions will apply and whenever appropriate, terms used in the singular will also include the plural and *vice versa.* It is to be understood that the terminology used herein is for the purpose of describing particular embodiments only and is not intended to be limiting.

Features, integers, characteristics, compounds described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments.

The term "comprise" and variations thereof, such as, "comprises" and "comprising" is generally used in the sense of include, that is, as "including, but not limited to", that is to say permitting the presence of one or more features or components.

The singular forms "a," "an," and "the" include plural referents unless the content clearly dictates otherwise.

The term "about" refers to a range of values ± 10% of a specified value. For example, the phrase "about 200" includes ± 10% of 200, or from 180 to 220.

The term "solid carrier" as used herein refers usually to a particle. Preferably the solid carrier is a monodisperse particle or a polydisperse particle, more preferably a monodisperse particle. The solid carrier usually comprises organic particles, inorganic particles, organic-inorganic particles, self-assembling organic particles, silica particles, gold particles, titanium particles and is preferably a silica particle, more preferably a silica nanoparticle (SNP). The particle size of the solid carrier is usually between 1 nm and 1000 µm, preferably between 10 nm and 100 µm, particularly about 50 nm.

The term "linker" or "cross-linker" which are used synonymously herein refers to any linking reagents containing groups, which are capable of binding to specific functional groups (e.g. primary amines, sulfhydryls, etc.). A linker in the context of the present invention usually connects the surface of the solid carrier with the branched-chain amino acid-degrading enzyme. For example, a linker may be immobilized on the surface of the solid carrier e.g. on the silica surface as a carrier material and then the branched-chain amino acid-degrading enzyme may be bound to an unoccupied binding-site of the linker. Alternatively, the linker may firstly bind to the branched-chain amino acid-degrading enzyme and then the linker bound to the branched-chain amino acid-degrading enzyme may bind with its unoccupied binding-site to the solid carrier. Various types of linkers are known in the art, including but not limited to straight or branched-chain carbon linkers, heterocyclic carbon linkers, peptide linkers, polyether linkers, and linkers that are known in the art as tags.

The term "protective layer" as used herein refers to a layer for protecting the functional properties of the branched-chain amino acid-degrading enzyme or fragment immobilized on the surface of the solid carrier. The protective layer of the present invention is usually built with building blocks at least part of which are monomers capable of interacting with both each other usually by covalent binding and the immobilized branched-chain amino acid-degrading enzyme usually by non-covalent binding. The protective layer is formed on the surface of the solid carrier to protect the branched-chain amino acid-degrading enzyme or the fragment thereof immobilized on the solid carrier. The protective layers are usually homogeneous layers where at least 50%, preferably at least 70%, more preferably at least 90% of the branched-chain amino acid-degrading enzyme or fragment therof are embedded in the protective layer.

The terms "branched-chain amino acid-degrading enzyme or a fragment thereof" or "enzyme or a fragment thereof that degrades branched-chain amino acids" are synonymously used herein and refer to enzymes that degrade the branched-chain amino acids leucine, isoleucine, and valine. Branched-chain amino acid-degrading enzymes are e.g. decarboxylases, such as leucine decarboxylase and valine decarboxylase that break down branched-chain amino acid e.g. leucine into an amine and carbondioxide. The term, "branched-chain amino acid-degrading enzyme or a fragment thereof" includes naturally occurring branched-chain amino acid-degrading enzymes or a fragment thereof and also includes artificially engineered branched-chain amino acid-degrading enzymes or a fragment thereof. Artificially engineered branched-chain amino acid-degrading enzymes or a fragment thereof are e.g. variants or functionally active fragments of the branched-chain amino acid-degrading enzyme. The terms "fragment of a branched-chain amino acid-degrading enzyme", "fragment thereof" in relation to the branched-chain amino acid-degrading enzyme and "functionally active fragment of the branched-chain amino acid-degrading enzyme" are thus used synonymously herein. By "variants or functionally active fragments thereof" in relation to the branched-chain amino acid-degrading enzyme of the present invention is meant that the fragment or variant (such as an analogue, derivative or mutant) is capable of exercising the same physiological function as the branched-chain amino acid-degrading enzyme. Such variants include naturally occurring allelic variants and non-naturally occurring variants. Additions, deletions, substitutions and derivatizations of one or more of the amino acids are contemplated so long as the modifications do not result in loss of functional activity of the fragment or variant. Preferably the functionally active fragment or variant has at least about 80% sequence identity more preferably at least about 90% sequence identity, even more preferably at least about 95% sequence identity, most preferably at least about 98% sequence identity to the relevant part of the branched-chain amino acid-degrading enzyme. A fragment of a branched-chain amino acid-degrading enzyme as defined herein does usually have the same functional properties as the branched-chain amino acid-degrading enzyme from which it is derived. A fragment of a branched-chain amino acid-degrading enzyme contains usually between 100 and 1000 amino acids, preferably between 300 and 800 amino acids, more preferably between 450 and 650 amino acids. The branched-chain amino acid-degrading enzyme or a fragment thereof of the present invention is preferably a leucine-degrading enzyme or a fragment thereof.

The term, "leucine-degrading enzyme or a fragment thereof" includes naturally occurring leucine-degrading enzymes or a fragment thereof and also includes artificially engineered leucine-degrading enzymes or a fragment thereof. Leucine-degrading enzymes are decarboxylases, such as leucine decarboxylase and valine decarboxylase that break down leucine into an amine and carbondioxide. Artificially engineered leucine-degrading enzymes or a fragment thereof are e.g. variants or functionally active fragments of the leucine-degrading enzyme. The terms "fragment of a leucine-degrading enzyme", "fragment thereof" in relation to the leucine-degrading enzyme and "functionally active fragment of the leucine-degrading enzyme" are thus used synonymously herein. By "variants or functionally active fragments thereof" in relation to the leucine-degrading enzyme of the present invention is meant that the fragment or variant (such as an analogue, derivative or mutant) is capable of exercising the same physiological function as the leucine-degrading enzyme. Such variants include naturally occurring allelic variants and non-naturally occurring variants. Additions, deletions, substitutions and derivatizations of one or more of the amino acids are contemplated so long as the modifications do not result in loss of functional activity of the fragment or variant. Preferably the functionally active fragment or variant has at least about 80% sequence identity more preferably at least about 90% sequence identity, even more preferably at least about 95% sequence identity, most preferably at least about 98% sequence identity to the relevant part of the leucine-degrading enzyme. A fragment of a leucine-degrading enzyme as defined herein does usually have the same functional properties as the leucine-degrading enzyme from which it is derived. A fragment of a leucine-degrading enzyme, in particular a fragment of leucine decarboxylase contains usually between 100 and 1000 amino acids, preferably between 300 and 800 amino acids, more preferably between 450 and 650 amino acids.

The term "partially embedded branched-chain amino acid-degrading enzyme" as used herein shall mean that the branched-chain amino acid-degrading enzyme is not fully covered by the protective layer, thus, the branched-chain amino acid-degrading enzyme is not fully embedded in the protective layer. In one embodiment less than 50% of the branched-chain amino acid-degrading enzyme of interest are covered by the protective layer, though typically more at least 70% will be covered, thus improving protection of the branched-chain amino acid-degrading enzyme. In a preferred embodiment, at least 70%, more preferably at least 80%, even more preferably at least 90%, most preferably at least 95% of the branched-chain amino acid-degrading enzyme of interest is covered by the protective layer. In another preferred embodiment, around 70% to around 95%, more preferrably around 80% to around 95%, even more preferably around 90% to around 95%, most preferably around 90% to around 95, 96, 97, 98 or 99 % of the branched-chain amino acid-degrading enzyme of interest are covered by the protective layer. In a particularly preferred embodiment, around 70%, particularly around 80%, more particularly around 90%, most particularly around 95% of the branched-chain amino acid-degrading enzyme of interest is covered by the protective layer. In a more particularly preferred embodiment, around 70%, particularly around 80%, more particularly around 90%, most particularly around 95% of the branched-chain amino acid-degrading enzyme of interest is covered by the protective layer, wherein the active site is not covered.

The term "fully embedded branched-chain amino acid-degrading enzyme" as used herein shall mean that the branched-chain amino acid-degrading enzyme of interest according to the invention is fully, i.e. 100% covered by the protective layer, i.e. that also the active site is covered.

The term "at least partially embedded branched-chain amino acid-degrading enzyme" as used herein shall mean that the branched-chain amino acid-degrading enzyme is at least partially embedded and may be fully embedded by the protective layer. Thus "at least partially embedded branched-chain amino acid-degrading enzyme" means that the protective layer covers from about 30% and 100% of the branched-chain amino acid-degrading enzyme or a fragment therof, preferably from about 50% to about 100%, more preferably from about 80% to about 100%, even more preferably from about 90% to about 100%, most preferably from about 95% to about 100 %, wherein the active site is preferably covered.

The term "functional constitutent" as used herein refers to a constituent which after being immobilized to the surface of the protective layer retains its characteristic, functional property. A functional constituent in the sense of the present invention is a polymer comprising repeat units wherein each repeat unit comprises at least one amino group and/or at least one thiol group.

The term "polymer comprising repeat units wherein each repeat unit comprises at least one amino group" as used herein refers to a polymer comprising a number of repeat units (monomers), whererin each repeat unit comprises at least one amino group. A preferred polymer comprises a number of repeat units (monomers), whererin each repeat unit contains one amino group, in particular one primary amino group.

The term "polymer comprising repeat units wherein each repeat unit comprises at least one thiol group" as used herein refers to a polymer comprising a number of repeat units (monomers), whererin each repeat unit comprises at least one thiol. A preferred polymer comprises a number of repeat units (monomers), whererin each repeat unit contains one thiol group.

The term "polycarbophil-cysteine conjugates" as used herein refers to conjugates which comprise cysteine covalently attached to polycarbophil. Such conjugates can be produced as referred in e.g. Bernkop-Schnurch and Thaler, 2000, Journal of Pharmaceutical Sciences 89(7):901-9.

The term "polylysine" as used herein refers to α-polylysine and or ε-polylysine (ε-poly-L-lysine, EPL), preferably ε-polylysine. α-polylysine is a synthetic polymer, which can be composed of either L-lysine or D-lysine. ε-polylysine (ε-poly-L-lysine, EPL) is typically produced as a homopolypeptide of approximately 25-30 L-lysine residues.

The term "polycysteine" as used herein can be composed of either L-cysteine or D-cysteine and is preferably composed of L-cysteine and comprises preferably between 2 and 30 cysteine residues, more preferably between 2 and 5 cysteine residues.

The term "polyglucosamin" as used herein refers to linear amino-polysaccharides composed of D-glucosamine and N-acetyl-D-glucosamine units linked by (1-4) glycosidic bonds. Polyglucosamine contains free amine (-NH2) groups and may be characterized by the proportion of N-acetyl-D-glucosamine units and D-glucosamine units, which is expressed as the degree of deacetylation (DDA) of the fully acetylated polymer chitin. A preferred polyglucosamin of the present invention is selected from the group consisting of chitin, chitosan, polyglucosaminoglycans, chondroitin, heparin, keratan and dermatan or a derivative thereof. Most preferred is a chitosan or a derivative thereof.

The term "chitosan or a derivative thereof" as used herein refers to a chitosan or chitosan derivative thereof including a salt thereof which has preferably a molecular weight of 2 000 Da or more, preferably in the range 25 000 - 2 000 000 Da and more preferably about 50 000 - 350 000 Da, most preferably about 50 000 - 190 000 Da or 190 000 - 310 000 Da. The term "derivative" in relation to chitosan includes ester, ether or other derivatives formed by reaction of acyl or alkyl groups with the OH groups. Examples are O-alkyl ethers of chitosan, O-acyl esters of chitosan. Suitable derivatives are given e.g. in G.A.E. Roberts, Chitin Chemistry, MacMillan Press Ltd, London, 1992. Suitable salts of chitosan include nitrates, phosphates, sulphates, xanthates, hydrochlorides, glutamates, lactates, acetates.

The term "branched-chain amino acid metabolism related diseases" as used herein refers to diseases which relate or are caused by an impairment or lack of metabolism (break down or degradation) of the three branched-chain amino acids (BCAAs) leucine, isoleucine and valine, in the body. Branched-chain amino acid metabolism related diseases comprise Maple Syrup Urine Disease (MSUD) and propionic acidemia.

The term "Maple Syrup Urine Disease (MSUD)" as used herein refers to a rare genetic disorder characterized by deficiency of an enzyme complex (branched-chain alpha-keto acid dehydrogenase) that is required to degrade (break down or metabolize) the three branched-chain amino acids (BCAAs) leucine, isoleucine and valine, in the body. The result of this metabolic failure is that all three BCAAs, along with a number of their toxic byproducts, (specifically their respective organic acids), all accumulate abnormally. In the classic, severe form of MSUD, plasma concentrations of the BCAAs begin to rise within a few hours of birth. MSUD comprises intermittent MSUD which is a mild form of MSUD where patients (when well) are asymptomatic with normal levels of branched-chain amino acids (BCAAs) but with catabolic stress are at risk of acute decompensation with ketoacidosis, which can lead to cerebral edema and coma if untreated.

The term "propionic acidemia" as used herein refers to a disease caused by a deficiency of propionyl-CoA carboxylase, a mitochondrial biotin-dependent enzyme, resulting in elevation of serum and urine propionic acid. There are several presentations including severe neonatal, chronic intermittent, and gradually progressive. Symptoms of chronic disease are failure to thrive, microcephaly, intellectual disability, spastic quadriplegia, athetosis, dystonia, chorea, myoclonus, seizures, optic neuropathy, and myopathy.

"In a first aspect the present invention provides a composition comprising a solid carrier, a branched-chain amino acid-degrading enzyme or a fragment thereof immobilized on the surface of the solid carrier, a protective layer to protect the branched-chain amino acid-degrading enzyme or a fragment thereof by embedding the branched-chain amino acid-degrading enzyme or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group.

The branched-chain amino acid-degrading enzyme or a fragment thereof can be immobilized on the surface of the solid carrier by non-covalent binding or covalent binding. Non-covalent binding includes p-p (aromatic) interactions, van der Waals interactions, H-bonding interactions, and electrostatic interactions like e.g. ionic interactions. Preferably, the branched-chain amino acid-degrading enzyme or fragment thereofis immobilized on the surface of the solid carrier by covalent binding or by covalent binding via a linker.

A solution of a branched-chain amino acid-degrading enzyme or a fragment thereof usually comprises the protein or a fragment thereof in a buffer solution. Buffers which can be used are usually phosphate, chloride, MES, MOPS, HEPES, PIPES, ACES or mixtures thereof. The solution may additionally contain non-ionic surfactants as described herein. A solution of a branched-chain amino acid-degrading enzyme or a fragment thereof can be prepared by e.g. dissolving the branched-chain amino acid-degrading enzyme or a fragment thereof in water to reconstitute the stock buffer of branched-chain amino acid-degrading enzyme or a fragment thereof.

In one embodiment the solid carrier is selected from the group of organic particles, inorganic particles, organic-inorganic particles, self-assembling organic particles, silica particles, gold particles, titanium particles and is preferably a silica particle, more preferably a silica nanoparticle (SNP). The particle size is usually measured by measuring the diameter of the particles and is usually between 1 nm and 1000 nm, preferably between 10 nm and 100 nm, particularly about 50 nm. In case the solid carrier is a monodisperse particle, the size is usually between 1 nm and 1000 nm, preferably between 10 nm and 100 nm, particularly about 50 nm. In case the solid carrier is a polydisperse particle, the size is usually between1 nm and 1000 µm, preferably between 10 nm and 100 µm, particularly between 50 nm and 50 µm. In one embodiment the composition comprises a solid carrier wherein the solid carrier comprises at least 15%, preferably at least 20%, in particular between 15% and 30%, more particular between 20% and 30 % immobilized branched-chain amino acid-degrading enzyme or fragment thereof per dry weight of the solid carrier.

Usually monodisperse particles or polydisperse particles, preferably monodisperse particles are used as solid carrier in the present invention. In a preferred embodiment the monodisperse particles are spherical monodisperse particles. In a further preferred embodiment, the polydisperse particles are non-spherical polydisperse particles.

The solid carrier is usually provided in suspension. Suspension of the solid carrier can be e.g. in water, buffer or non-ionic surfactants or mixtures thereof, preferably in mixtures of water and non-ionic surfactants. Non-ionic surfactants used are usually polysorbate 80 (PS80). Buffers which can be used in the method of the present invention are phosphate, piperazine-N,N'-bis(2-ethanesulfonic acid), 2-Hydroxy-3-morpholinopropanesulfonic acid, N,N-bis[2-hydroxyethyl]-2-aminoethanesulfonic acid), (3-(N-morpholino)propanesulfonic acid), 2-[[1,3-dihydroxy-2-(hydroxymethyl)propan-2-yl]amino]ethanesulfonic acid, 4-(2-hydroxyethyl)-1-piperazineethanesulfonic acid), 3-(N,N-Bis[2-hydroxyethyl]amino)-2-hydroxypropanesulfonic acid, N,N-Bis(2-hydroxyethyl)-3-amino-2-hydroxypropanesulfonic acid, N-[Tris(hydroxymethyl)methyl]glycine, Diglycine, 4-(2-Hydroxyethyl)-1-piperazinepropanesulfonic acid, N,N-Bis(2-hydroxyethyl)glycine, N-[Tris(hydroxymethyl)methyl]-3-aminopropanesulfonic acid, N-(1,1-Dimethyl-2-hydroxyethyl)-3-amino-2-hydroxypropanesulfonic acid.

In one embodiment the surface of the solid carrier is modified to introduce a molecule or functional chemical group as anchoring point i.e. as anchoring point for the branched-chain amino acid-degrading enzyme or for the linker connecting the branched-chain amino acid-degrading enzyme to the solid carrier. Preferably, said anchoring point is an amine functional chemical group or moiety. As a non-limiting example, an amino-modified surface of the solid carrier e.g. an amino-modified silica surface may be used as modified solid carrier. Such an amino-modified surface of the solid carrier may be obtained by reacting a solid carrier having a silica surface with an amino silane, e.g. with APTES. Thus in a preferred embodiment, the solid carrier is a solid carrier having a silica surface with an amino-modified surface, more preferably a solid carrier obtained by reacting the solid carrier having a silica surface with an amino silane, e.g. with APTES. Such a modified carrier may form an amide linkage between the branched-chain amino acid-degrading enzyme and the amine group at the surface of the carrier material or an amide linkage between the linker and the amine group at the surface of the carrier material. In one embodiment the introduced molecule or functional chemical group as anchoring point is homogeneously distributed on the surface of the solid carrier.

In some embodiments the protective layer has a defined thickness of about 1 to about 200 nm, usually 1 to about 100 nm, preferably about 1 to about 50nm, more preferably about 1 to about 25 nm, even more preferably about 1 to about 20 nm, in particular about 1 to about 15 nm. The most preferred defined thickness is about 1 to about 10 nm. In some embodiments the layer has a defined thickness of about 5 to about 100 nm, preferably about 5 to about 50 nm, more preferably about 5 to about 25 nm, even more preferably about 5 to about 20 nm, in particular about 5 to about 15 nm. The most preferred defined thickness is about 5 to about 10 nm. The protective layer is usually porous and the pore size is between 1 and 20 nm, preferably between 1 and 5 nm. The protective layer thickness can be measured, by using a microscope such as scanning electron microscope (SEM), transmission electron microscopy (TEM), scanning probe microscopy (SPM), light scattering methods or by ellipsometry.

In one embodiment, the branched-chain amino acid-degrading enzyme or fragment thereof is partially embedded by the protective layer. In a preferred embodiment the branched-chain amino acid-degrading enzyme or fragment thereof is at least partially embedded by the protective layer. In a more preferred embodiment the branched-chain amino acid-degrading enzyme or fragment thereofis fully embedded by the protective layer.

In one embodiment, the protective layer embeds the solid carrier and embeds the branched-chain amino acid-degrading enzyme or fragment thereof thereof immobilized on the surface of the solid carrier. In one embodiment, the functional constituent immobilized on the surface of the protective layer, is not embedded by the protective layer. Preferably, the protective layer fully embeds the solid carrier and fully embeds the branched-chain amino acid-degrading enzyme or fragment thereof immobilized on the surface of the solid carrier. More preferably, the protective layer fully embeds the solid carrier and fully embeds the branched-chain amino acid-degrading enzyme or fragment thereof immobilized on the surface of the solid carrier and the functional constituent immobilized on the surface of the protective layer is not embedded by the protective layer. If the protective layer fully embeds the solid carrier and fully embeds the branched-chain amino acid-degrading enzyme or fragment thereof immobilized on the surface of the solid carrier, the branched-chain amino acid-degrading enzyme or fragment thereof is fully, i.e. 100% covered by the protective layer, i.e. that also the active site is covered and the solid carrier is fully, i.e. 100% covered by the protective layer.

In one embodiment the branched-chain amino acid-degrading enzyme or a fragment thereof of the present invention is a branched-chain amino acid decarboxylase or a fragment thereof. In a preferred embodiment the branched-chain amino acid-degrading enzyme or a fragment thereof of the present invention is a leucine-degrading enzyme or a fragment thereof. In a more preferred embodiment the leucine-degrading enzyme or a fragment thereof is selected from the group consisting of leucine decarboxylase or a fragment thereof and valine decarboxylase or a fragment thereof or mixtures thereof, and is even more preferably leucine decarboxylase or a fragment thereof, in particular leucine decarboxylase of Gene model 853 (NCBI) (Gene Ldc1) from Mus musculus (Mouse) (Uniprot Q3UNZ2). Thus the composition also comprises more than one e.g. two different branched-chain amino acid-degrading enzymes or fragments thereof immobilized on the surface of the solid carrier and embedded. When more than one branched-chain amino acid-degrading enzyme or a fragment thereof is used, preferably two different branched-chain amino acid-degrading enzyme s, more preferably leucine decarboxylase or a fragment thereof and valine decarboxylase or a fragment thereof are used.

In a particular preferred embodiment the branched-chain amino acid-degrading enzyme or a fragment thereof is selected from the group consisting of leucine decarboxylase or a fragment thereof and valine decarboxylase or a fragment thereof. Most preferred is leucine decarboxylase or a fragment thereof.

The composition of the present invention is usually produced in a reaction vessel like a reactor. The formation of the protective layer is usually carried out by forming the respective protective layer by building blocks, wherein the building blocks build the protective layer in a polycondensation reaction. The polycondensation can be effected in different solvents, preferably in aqueous solution. Polycondensation can be easily controlled and stopped if appropriate, allowing achievement of a defined thickness of the protective layer. The choice of the building blocks, which can be used to build the protective layer, may depend on the known structure of the branched-chain amino acid-degrading enzyme in order to adapt the affinity of the protective layer according to optimal and/or desired parameters. As building blocks for the protective layer usually structural building blocks and protective building blocks are used to build the protective layer. Structural building blocks which can be used are e.g. tetraethylorthosilicate (designated herein as "TEOS" or "T"). Protective building blocks which can be used are e.g. 3-Aminopropyltriethoxysilane (designated herein as "APTES" or "A"), Propyltriethyoxysilane (designated herein as "PTES" or P"), Isobutyltriethoxysilane (designated as "IBTES"), Hydroxymethyltriethoxysilane (designated herein as "HTMEOS" or H), Benzyltriethoxysilane (designated herein as "BTES"), Ureidopropyltriethoxysilane (designated as "UPTES"), or Carboxyethyltriethoxysilane (designated herein as "CETES"). Structural building blocks are usually precursors of inorganic silica, capable of forming 4 covalent bonds in the layer formed. Protective building blocks are usually organosilanes, bearing an organic moiety endowed with the ability to interact with the branched-chain amino acid-degrading enzymes. Preferred structural building blocks are tetravalent silanes, in particular tetra-alkoxy-silanes. Preferred protective building blocks are trivalent silanes, in particular tri-alkoxy-silanes. More preferred structural building blocks are mixtures of tetravalent silanes and trivalent silanes, in particular mixtures of tetra-alkoxy-silanes and tri-alkoxy-silanes. Even more preferred structural building blocks are selected from the group consisting of tetraethylorthosilicate, tetra-(2-hydroxyethyl)silane, and tetramethylorthosilicate. Even more preferred protective building blocks are selected from the group consisting of carboxyethylsilanetriol, benzylsilanes, propylsilanes, isobutylsilanes, n-octylsilanes, hydroxysilanes, bis(2-hydroxyethyl)-3 -aminopropylsilanes, aminopropylsilanes, ureidopropylsilanes, (N-Acetylglycyl)-3-aminopropylsilanes, hydroxy(polyethyleneoxy)propyl]triethoxysilanes, in particular selected from benzyltriethoxysilane, propyltriethoxysilane, isobutyltriethoxysilane, n-octyltriethoxysilane, hydroxymethyltriethoxysilane, bis(2-hydroxyethyl)-3 -aminopropyltriethoxysilane, 3-Aminopropyltriethoxysilane, ureidopropyltriethoxysllane, (N-Acetylglycyl)-3-aminopropyltriethoxysilane, or selected from benzyltrimethoxysflane, propyltrimethoxysilane, isobutylimethoxysilane, n-octyltrimethoxysilane, hydroxymethyltrimethoxysilane, bis(2-hydroxyethyl)-3 -aminopropyltrimethoxysilane, aminopropyltrimethoxysilane, ureidopropyltrimethoxysilane (N-Acetylglycyl)-3 -aminopropyltrimethoxysilane or selected from benzyltrihydroxyethoxysilane, propyltrihydroxyethoxysilane, isobutyltrihydroxyethoxysilane, n-octyltrihydroxyethoxysilane, hydroxymefilyltrihydroxyethoxysilane, bis(2-hydroxyethyl)-3 - aminopropyltrihydroxyethoxysilane, aminopropyltrihydroxyethoxysilane, Ureidopropyltrihydroxyethoxysilane (N-Acetylglycyl)-3-aminopropyltrihydroxymethoxysilane.

Particular preferred building blocks are TEOS as structural building block and APTES, PTES, and/or HTMEOS, preferably APTES as protective building block. In particular TEOS as structural building block and APTES as protective building block are used to build the protective layer.

The reaction time of the building blocks with the solid carrier depends on the length of the linker, if a linker is used, and the size of the branched-chain amino acid-degrading enzyme. The reaction is usually carried out for a time period of between 0.5 to 10 hours, preferably between 1 and 5 hours, more preferably between 1 and 4 hours, even more preferably between 2 and 4 hours, preferably in aqueous solution and preferably at room temperature of about 5 to about 25 °C or at about 20 °C. The formation of the protective layer can be stopped by actively stopping the polycondensation reaction e.g by removing the non-reacted building blocks e.g. by a washing step or by self-stopping of the polycondensation reaction caused by a limited amount of buidling blocks.

In a furthermore preferred embodiment the branched-chain amino acid-degrading enzyme is immobilized on the solid carrier by at least partly modifying the surface of the solid carrier by introducing a molecule as anchoring point as described supra for the branched-chain amino acid-degrading enzyme and by using a linker, preferably a cross-linker binding to the anchoring point and the branched-chain amino acid-degrading enzyme.

In one embodiment the introduced molecule as anchoring point and/or the linker are homogeneously distributed on the surface of the solid carrier.

In a preferred embodiment the cross-linker is selected from the group consisting of glutaraldehyde, disuccinimidyl tartrate, bis[sulfosuccinimidyl]suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, activated sulfhydrils, sulfhydryl-reactive 2-pyridyldithiol, BSOCOES (Bis[2-(succinimidooxycarbonyloxy)ethyl]sulfone), DSP (Dithiobis[succinimidyl]propionate]), DTSSP (3,3 '-Dithiobis[sulfosuccinimidyl]propionate]), DTBP (Dimethyl 3,3 '-dithiobispropionimidate 2 HCl), DST (Disuccinimidyl tartarate), Sulfo-LC-SMPT (4-Sulfosuccinimidyl-6-methyl-a-(2-pyridyldithio)toluamido]hexanoate)), SPDP (N-Succinimidyl 3-(2-pyridyldithio)-propionate), LC-SPDP (Succinimidyl 6-(3-[2-pyridyldithio]-propionamido)hexanoate), SMPT (4-Succinimidyloxycarbonyl-methyl-a-[2-pyridyldithio]toluene), DPDPB (1,4-Di-[3'-(2'-pyridyldithio)-propionamido]butane), DTME (Dithio-bismaleimidoethane), BMDB (1,4 bismaleimidyl-2,3-dihydroxybutane). More preferably said cross-linker is selected from glutaraldehyde, disuccinimidyl tartrate, disuccinimidyl suberate, bis[sulfosuccinimidyl] suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, activated sulfhydrils (e.g. suflhydryl-reactive 2-pyridyldithio). In a more preferred embodiment the cross-linker is selected from the group consisting of glutaraldehyde, disuccinimidyl tartrate, bis[sulfosuccinimidyl]suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, BSOCOES (Bis[2-(succinimidooxycarbonyloxy)ethyl]sulfone), DSP (Dithiobis[succinimidyl]propionate]), DTSSP (3,3 '-Dithiobis[sulfosuccinimidyl]propionate]), DTBP (Dimethyl 3,3 '-dithiobispropionimidate-2 HCl), DST (Disuccinimidyl tartarate), BMDB (1,4 bismaleimidyl-2,3-dihydroxybutane). More preferably said cross-linker is selected from glutaraldehyde, disuccinimidyl tartrate, disuccinimidyl suberate, bis[sulfosuccinimidyl] suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, activated sulfhydrils (e.g. suflhydryl-reactive 2-pyridyldithio). Most preferred is glutaraldehyde.

After the protective layer has been formed, the solid carrier comprising the branched-chain amino acid-degrading enzyme and the protective layer can be stored. Storing is usually accomplished e.g. by washing the composition formed e.g. with a buffer and storing it suspended or solved in that buffer for a desired time period. In a preferred embodiment the solid carrier comprising the branched-chain amino acid-degrading enzyme and the protective layer is stored at a constant temperature between 2 to 25 °C. In a further preferred embodiment, the solid carrier comprising the branched-chain amino acid-degrading enzyme and the protective layer is stored 5 to 48 hours, preferably 10 to 30 hours. More preferably the solid carrier comprising the branched-chain amino acid-degrading enzyme and the protective layer is stored at a constant temperature between 2 to 25 °C, preferably at room temperature for 10 to 30 hours.

In one embodiment, the functional constituent binds to mucus.

In one embodiment a polymer comprising repeat units wherein each repeat unit comprises at least one amino group and/or at least one thiol group is a polymer comprising repeat units wherein each repeat unit comprises at least one amino group.

In one embodiment a polymer comprising repeat units wherein each repeat unit comprises at least one amino group and/or at least one thiol group is a polymer comprising repeat units wherein each repeat unit comprises at least one thiol group.

In one embodiment a polymer comprising repeat units wherein each repeat unit comprises at least one amino group and/or at least one thiol group is selected from the group consisting of a polyglucosamin, a polymerized silane-PEG-NH2 and a polymerized silane comprising an amino group. In a preferred embodiment the polymer comprising repeat units wherein each repeat unit comprises at least one amino group and/or at least one thiol group, is selected from the group consisting of a polyglucosamin, a polymerized silane-PEG-NH2 and polymerized APTES.

In a more preferred embodiment the polymer comprising repeat units wherein each repeat unit comprises at least one amino group and/or at least one thiol group is selected from the group consisting of a polyglucosamin selected from the group consisting of chitin, chitosan, polyglucosaminoglycans, chondroitin, heparin, keratan and dermatan or a derivative thereof; a polymerized silane-PEG-NH2; and a polymerized silane comprising an amino group, preferably a polymerized APTES. In an even more preferred embodiment the polymer comprising repeat units wherein each repeat unit comprises at least one amino group and/or at least one thiol group, is a polyglucosamin, preferably a polyglucosamin selected from the group consisting of chitin, chitosan, polyglucosaminoglycans, chondroitin, heparin, keratan and dermatan or a derivative thereof, more preferably a chitosan or a derivative thereof. The most preferred polymer comprising repeat units wherein each repeat unit comprises at least one amino group and/or at least one thiol group is a chitosan or a derivative thereof.

A preferred polyglucosamin of the present invention is selected from the group consisting of chitin, chitosan, polyglucosaminoglycans, chondroitin, heparin, keratan and dermatan or a derivative thereof. Most preferred is a chitosan or a derivative thereof. A preferred silane-PEG-NH2 of the polymerized silane-PEG-NH2 is selected from the group consisting of silane-PEG4-NH2, silane-PEG2000-NH2, and silane-PEG5000-NH2. A preferred polymerized silane comprising an amino group is selected from the group consisting of APTES, amino-butyl-TES, amino-pentyl-TES, amino-hexyl-TES, amino-heptyl-TES, and amino-octyl-TES, and is in particular APTES.

In a further embodiment a polymer comprising repeat units wherein each repeat unit comprises at least one amino group and/or at least one thiol group is selected from the group consisting of a polyglucosamin, a polymerized silane-PEG-NH2, a polymerized silane comprising an amino group, a polymerized silane comprising a thiol group, a polycarbophil-cysteine conjugate, a polymerized silane-PEG-thiol and a polycysteine. In a further more preferred embodiment the polymer comprising repeat units wherein each repeat unit comprises at least one amino group and/or at least one thiol group is selected from the group consisting of a polyglucosamin selected from the group consisting of chitin, chitosan, polyglucosaminoglycans, chondroitin, heparin, keratan and dermatan or a derivative thereof; a polymerized silane-PEG-NH2; a polymerized silane comprising a thiol group, preferably a polymerized MPTS; a polycarbophil-cysteine conjugate; a polymerized silane-PEG-thiol; and a polycysteine. In an even more preferred embodiment the polymer comprising repeat units wherein each repeat unit comprises at least one amino group and/or at least one thiol group, is a polyglucosamin or a polymerized silane comprising a thiol group, preferably a polyglucosamin selected from the group consisting of chitin, chitosan, polyglucosaminoglycans, chondroitin, heparin, keratan and dermatan or a derivative thereof, more preferably a chitosan or a derivative thereof or a polymerized silane comprising a thiol group, a polycarbophil-cysteine conjugate, and a polymerized silane-PEG-thiol, preferably a polymerized silane comprising a thiol group.

In a particular embodiment, the polymer comprising repeat units wherein each repeat unit comprises at least one amino group and/or at least one thiol group, is selected from the group consisting of chitin, chitosan, polyglucosaminoglycans, chondroitin, heparin, keratin, dermatan or a derivative thereof in particular chitosan or a derivative thereof, a polymerized silane-PEG-NH2 selected from the group consisting of polymerized silane-PEG4-NH2, polymerized silane-PEG2000-NH2, polymerized silane-PEG5000-NH2, a polymerized silane comprising an amino group which is preferably polymerized APTES and a polymerized silane comprising a thiol group, which is preferably polymerized MPTS.

In one embodiment a polymer comprising repeat units wherein each repeat unit comprises at least one amino group and/or at least one thiol group is selected from the group consisting of a polyglucosamin, a polymerized silane-PEG-NH2, a polymerized silane comprising an amino group and a polymerized silane comprising a thiol group. In a preferred embodiment the polymer comprising repeat units wherein each repeat unit comprises at least one amino group and/or at least one thiol group, is selected from the group consisting of a polyglucosamin, a polymerized silane-PEG-NH2, polymerized APTES and polymerized MPTS.

In a more preferred embodiment the polymer comprising repeat units wherein each repeat unit comprises at least one amino group and/or at least one thiol group is selected from the group consisting of a polyglucosamin selected from the group consisting of chitin, chitosan, polyglucosaminoglycans, chondroitin, heparin, keratan and dermatan or a derivative thereof; a polymerized silane-PEG-NH2; a polymerized silane comprising an amino group, preferably a polymerized APTES; and a polymerized silane comprising a thiol group, preferably polymerized MPTS. In a particular embodiment, the polymer comprising repeat units wherein each repeat unit comprises at least one amino group and/or at least one thiol group, is selected from the group consisting of chitin, chitosan, polyglucosaminoglycans, chondroitin, heparin, keratin, dermatan or a derivative thereof, most particular chitosan or a derivative thereof.

In one embodiment a polymer comprising repeat units wherein each repeat unit comprises at least one thiol group is selected from the group consisting of a polymerized silane comprising a thiol group, a polycarbophil-cysteine conjugate, a polymerized silane-PEG-thiol and a polycysteine, and is preferably selected from the group consisting of a polymerized silane comprising a thiol group, a polycarbophil-cysteine conjugate, and a polymerized silane-PEG-thiol, and is more preferably a polymerized silane comprising a thiol group, and is most perferably polymerized MPTS. In one embodiment a polymerized silane comprising a thiol group is preferably polymerized MPTS.

In one embodiment 5% to 100%, preferably 10% to 100%, more preferably 50% to 100%, of the surface of the protective layer is covered with a polymer comprising repeat units wherein each repeat unit comprises at least one amino group and/or at least one thiol group.

In one embodiment the functional constituent is immobilized on the surface of the protective layer by binding, preferably covalent binding. In a preferred embodiment the functional constituent is immobilized on the surface of the protective layer by non-covalent binding, preferably by electrostatic interactions. In a more preferred embodiment the polymer comprising repeat units wherein each repeat unit comprises at least one amino group and/or at least one thiol group is immobilized on the surface of the protective layer by covalent binding.

In one embodiment the functional constituent is immobilized on the surface of the protective layer using a spacer binding to the surface of the protective layer and the functional constituent. Thus in one embodiment the present invention comprises a composition comprising a solid carrier, a branched-chain amino acid-degrading enzyme or a fragment thereof immobilized on the surface of the solid carrier, a protective layer to protect the branched-chain amino acid-degrading enzyme or a fragment thereof by embedding the branched-chain amino acid-degrading enzyme or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group, wherein the functional constituent is immobilized on the surface of the protective layer by a spacer. Examples of such a spacer include a polyethylene such as PEG4, PEG2000, PEG5000. A functional constituent immobilized on the surface of the protective layer, by a spacer is usually produced by firstly reacting the spacer with the functional constituent, so that the spacer binds to the functional constituent and then the functional constituent bound to the spacer is reacted with the the surface of the protective layer.

The immobilization of the functional constituent to the surface of the protective layer is usually carried out in a reaction vessel like a reactor by suspending the solid carrier carrying the branched-chain amino acid-degrading enzyme embedded in a protective layer as described supra in e.g. in water, buffer or non-ionic surfactants or mixtures thereof, preferably in mixtures of water and non-ionic surfactants. Non-ionic surfactants used are usually polysorbate 80 (PS80). The functional component is then added to the suspension to react usually under stirring with the surface of the protetctive layer to immobilize the functional constitutent on the surface of the protective layer. Ususally such obtained composition is washed and resuspended into water, buffer or non-ionic surfactants or mixtures thereof. Immobilization takes place by non-covalent binding e.g. electrostatic binding or by covalent binding of the functional constituent. The functional constituent may be immobilized by chemically modifying the surface of the protective layer and the functional constituent using e.g. "click chemistry" such as copper-catalyzed click chemistry (Copper-catalysed azide-alkyne cycloaddition, see e.g. Kolb et al. (2001) Angew. Chem. 40(11)2004-2021) or by copper free click chemistry (Wittig G, A Chem Ber, 1961, 94, 3260) ., e.g. the solid carrier carrying the branched-chain amino acid-degrading enzyme embedded in a protetctive layer as described supra is first reacted with a reactive compound like an ethynyl compound and the functional constituent is modified by adding a reactive compound e.g.an azide residue and then both components are reacted to immobilize the functional constituent on the surface of the protective layer.

A preferred composition comprises a solid carrier, whereby the solid carrier is a silica nanoparticle (SNP), a branched-chain amino acid-degrading enzyme or a fragment thereof immobilized on the surface of the solid carrier, wherein the branched-chain amino acid-degrading enzyme or a fragment thereof is a leucine decarboxylase or a fragment thereof, a protective layer to protect the branched-chain amino acid-degrading enzyme or a fragment thereof by embedding the branched-chain amino acid-degrading enzyme or a fragment thereof, wherein the protective layer comprises tetravalent silanes and trivalent silanes; and a functional constituent immobilized on the surface of the protective layer, wherein the functional constituent immobilized on the surface of the protective layer is a polymer comprising repeat units wherein each repeat unit comprises at least one amino group, preferably wherein the functional constituent immobilized on the surface of the protective layer is chitosan.

In a further aspect the present invention provides the composition as described supra for use as a medicament.

In a further aspect the present invention provides the composition for use in a method for the prevention, delay of progression or treatment of branched-chain amino acid metabolism related diseases, preferably for the prevention, delay of progression or treatment of Maple Syrup Urine Disease (MSUD) or proprionic academia, most preferably for the prevention, delay of progression or treatment of Maple Syrup Urine Disease (MSUD). Also provided is the use of the composition as described herein for the manufacture of a medicament for the prevention, delay of progression or treatment of branched-chain amino acid metabolism related diseases in a subject. Also provided is the use of the composition as described herein for the prevention, delay of progression or treatment of branched-chain amino acid metabolism related diseases in a subject. Also provided is a method for the prevention, delay of progression or treatment of branched-chain amino acid metabolism related diseasesin a subject, comprising administering to said subject a therapeutically effective amount of the composition as described herein.

A composition according to the invention is preferably a pharmaceutical composition and comprises a therapeutically effective amount of the composition as described herein and one or more suitable pharmaceutically acceptable carrier. A pharmaceutical composition according to the invention is suitable for oral administration to a subject. If not indicated otherwise, a pharmaceutical composition according to the invention is prepared in a manner known per se.

An exemplary treatment regime entails administration once daily, twice daily, three times daily, every second day, twice per week, once per week. The composition, e.g. the pharmaceutical composition of the invention is usually administered on multiple occasions. Intervals between single dosages can be, for example, less than a day, daily, every second day, twice per week, or weekly. The composition, e.g. the pharmaceutical composition of the invention may be given as a continous uninterrupted treatment. The composition, e.g. the pharmaceutical composition of the invention may also be given in a regime in which the subject receives cycles of treatment interrupted by a drug holiday or period of non-treatment.

Thus, the composition, e.g. the pharmaceutical composition of the invention may be administered according to the selected intervals above for a continuous period of one week or a part thereof, for two weeks, for three weeks for four weeks, for five weeks or for six weeks and then stopped for a period of one week, or a part thereof, for two weeks, for three weeks, for four weeks, for five weeks, or for six weeks.

The composition, e.g. the pharmaceutical composition of the present invention may conveniently be administered in unit dosage forms. Units ("U") of enzyme activity can be described in terms of weight or mass of substrate hydrolyzed per unit time. Units ("U") can be described in terms of umol substrate converted per minute (or umol/min).

The expression "effective amount" or "therapeutically effective amount" as used herein refers to an amount capable of invoking one or more of the desired effects in a subject receiving the composition of the present invention. Determination of a therapeutically effective amount is well within the capability of those skilled in the art, especially in light of the detailed disclosure provided herein.

The terms "treatment"/"treating" as used herein includes: (1) delaying the appearance of clinical symptoms of the state, disorder or condition developing in an animal, particularly a mammal and especially a human, that may be afflicted with or predisposed to the state, disorder or condition but does not yet experience or display clinical or subclinical symptoms of the state, disorder or condition; (2) inhibiting the state, disorder or condition (e.g. arresting, reducing or delaying the development of the disease, or a relapse thereof in case of maintenance treatment, of at least one clinical or subclinical symptom thereof); and/or (3) relieving the condition (i.e. causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

As used herein, "delay of progression" means increasing the time to appearance of a symptom of e.g. treatment of branched-chain amino acid metabolism related diseases or a mark associated with e.g. treatment of branched-chain amino acid metabolism related diseasesor slowing the increase in severity of a symptom of branched-chain amino acid metabolism related diseases. Further, "delay of progression" as used herein includes reversing or inhibition of disease progression. "Inhibition" of disease progression or disease complication in a subject means preventing or reducing the disease progression and/or disease complication in the subject.

Preventive treatments comprise prophylactic treatments. In preventive applications, the pharmaceutical combination of the invention is administered to a subject suspected of having, or at risk for developing the above mentioned diseases or disorders e.g. treatment of branched-chain amino acid metabolism related diseases. In therapeutic applications, the pharmaceutical combination is administered to a subject such as a patient already suffering from the above mentioned diseases or disorders e.g. treatment of branched-chain amino acid metabolism related diseases, in an amount sufficient to cure or at least partially arrest the symptoms of the disease. Amounts effective for this use will depend on the severity and course of the disease, previous therapy, the subject's health status and response to the drugs, and the judgment of the treating physician.

In the case wherein the subject's condition does not improve, the pharmaceutical combination of the invention may be administered chronically, which is, for an extended period of time, including throughout the duration of the subject's life in order to ameliorate or otherwise control or limit the symptoms of the subject's disease or condition.

In the case wherein the subject's status does improve, the pharmaceutical combination may be administered continuously; alternatively, the dose of drugs being administered may be temporarily reduced or temporarily suspended for a certain length of time (i.e., a "drug holiday"). Once improvement of the patient's condition has occurred, a maintenance dose of the pharmaceutical combination of the invention is administered if necessary. Subsequently, the dosage or the frequency of administration, or both, is optionally reduced, as a function of the symptoms, to a level at which the improved disease is retained.

In a further aspect the present invention provides a method of producing a composition as described supra, e.g. a composition comprising a solid carrier, a branched-chain amino acid-degrading enzyme or a fragment thereof immobilized on the surface of the solid carrier, a protective layer to protect the branched-chain amino acid-degrading enzyme or a fragment thereof by embedding the branched-chain amino acid-degrading enzyme or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group; the method
comprising the following steps:
(a) providing a solid carrier;
(b) immobilizing a branched-chain amino acid-degrading enzyme or a fragment thereof on the solid carrier;
(c) forming a protective layer on the surface of the solid carrier to protect the branched-chain amino acid-degrading enzyme or the fragment thereof immobilized on the solid carrier;
(d) immobilizing a functional constituent on the surface of the protective layer, wherein the functional constituent immobilized on the surface of the protective layer is a polymer comprising repeat units wherein each repeat unit comprises at least one amino group and/or at least one thiol group.

Step (a) is usually carried out by providing the solid carrier in suspension in water, non-ionic surfactants or a buffer or mixtures thereof, preferably in suspension in water and/or non-ionic surfactants, more preferably in suspension in water and/or non-ionic surfactants wherein no buffer is present in the suspension, even more preferably in suspension in mixtures of water and non-ionic surfactants in particular in suspension in mixtures of water and non-ionic surfactants wherein no buffer is present in the suspension. The suspension can be stirred e.g at 400 rpm, 20°C for 30 min. The immobilization of the branched-chain amino acid-degrading enzyme on the solid carrier in step b) of the present method is usually carried out by adding a solution of the branched-chain amino acid-degrading enzyme to the suspension of the solid carrier. Preferably a linker to connect the solid carrier with the branched-chain amino acid-degrading enzyme is added to the suspension of the solid carrier prior to adding the solution of the branched-chain amino acid-degrading enzyme to the suspension of the solid carrier. In a preferred embodiment the immobilization of the branched-chain amino acid-degrading enzyme on the solid carrier is carried out by providing a suspension of the solid carrier and adding a solution of the branched-chain amino acid-degrading enzyme, wherein the suspension with the added solution of the branched-chain amino acid-degrading enzyme is incubated to allow the enzyme to bind on the surface of the solid carrier. In a more preferred embodiment the immobilization of the branched-chain amino acid-degrading enzyme or a fragment thereof on the solid carrier in step b) is carried out by i) adding a linker to the solid carrier provided in step (a), preferably adding a linker to a suspension of the solid carrier provided in step a), and ii) adding the branched-chain amino acid-degrading enzyme or a fragment thereof, preferably adding a solution of the branched-chain amino acid-degrading enzyme or a fragment thereof, to the solid carrier and the linker or to the suspension comprising the solid carrier and the linker, wherein the linker connects the solid carrier with the branched-chain amino acid-degrading enzyme or a fragment thereof. In one embodiment, a building block of the protective layer, preferably a monomer of a building block of the protective layer, more preferably an organosilane, even more preferably a triethoxysilane, in particular APTES, is added to the solid carrier and the linker or to the suspension comprising the solid carrier and the linker, prior to adding the solution of the branched-chain amino acid-degrading enzyme or a fragment thereof. In a preferred embodiment the surface of the solid carrier is at least partly modified to improve immobilization of the branched-chain amino acid-degrading enzyme on the solid carrier. In particular, the surface of the solid carrier is at least partly modified before the branched-chain amino acid-degrading enzyme is immobilized. The surface of the solid carrier can be at least partly modified by adding a molecule as anchoring point for the branched-chain amino acid-degrading enzyme to the surface of the solid carrier as described supra.

The suspension comprising the solid carrier is usually incubated after each addition step described above to allow a reaction between e.g. the solid carrier and the molecule as anchoring point, the solid carrier and the linker and, the solid carrier comprising the linker and the branched-chain amino acid-degrading enzyme or a fragment thereof, respectively, so that the branched-chain amino acid-degrading enzyme or a fragment thereof connects the solid carrier, preferably the surface of the solid carrier, with the branched-chain amino acid-degrading enzyme or a fragment thereof via the linker, preferably by covalent binding, thereby immobilizing the branched-chain amino acid-degrading enzyme or a fragment thereof on the solid carrier.

In one embodiment in step (b) the branched-chain amino acid-degrading enzymeor a fragment thereof is immobilized on the solid carrier by connecting the solid carrier with the branched-chain amino acid-degrading enzymeor a fragment thereof via a linker, preferably by connecting the solid carrier with the branched-chain amino acid-degrading enzyme or a fragment thereof via a linker, wherein the solid carrier is connected with the branched-chain amino acid-degrading enzyme or a fragment thereof by covalent binding between the linker and the solid carrier and between the linker and the branched-chain amino acid-degrading enzyme or a fragment thereof. Preferably in step b), i) a linker is added to the solid carrier provided in step (a), and ii) the branched-chain amino acid-degrading enzyme or a fragment thereof is added to the solid carrier and the linker, wherein the linker connects the solid carrier with the branched-chain amino acid-degrading enzyme or a fragment thereof. The linker used is as described supra and connects the surface of the solid carrier with the branched-chain amino acid-degrading enzyme by preferably covalent binding. More preferably the linker is added to the solid carrier in step (b), in a molar excess to the branched-chain amino acid-degrading enzyme or a fragment thereof, preferably the linker is added to the solid carrier in step (b), in a 1 fold to 1000 fold molar excess to the branched-chain amino acid-degrading enzymeor a fragment thereof, more preferably the linker is added to the solid carrier in step (b), in a 2 fold to 300 fold molar excess to the branched-chain amino acid-degrading enzyme or a fragment thereof, even more preferably the linker is added to the solid carrier in step (b), in a 4 fold to 250 fold molar excess to the branched-chain amino acid-degrading enzyme or a fragment thereof, in particular the linker is added to the solid carrier in step (b), in a 25 fold molar excess to the branched-chain amino acid-degrading enzyme or a fragment thereof.

In a preferred embodiment the linker which has not connected the solid carrier with the branched-chain amino acid-degrading enzyme or a fragment thereof in step (b), is present during formation of a protective layer on the surface of the solid carrier in step (c). In a more preferred embodiment the linker which has not connected the solid carrier with the branched-chain amino acid-degrading enzyme or a fragment thereof in step (b), or a part thereof, covalently binds the protective layer to the branched-chain amino acid-degrading enzyme or the fragment thereof in step (c). In a furthermore preferred embodiment the linker which has not connected the solid carrier with the branched-chain amino acid-degrading enzyme or a fragment thereof in step (b) is not removed in step (b) or step (c) or in between step (b) and (c). In a particular embodiment the linker which has not connected the solid carrier with the branched-chain amino acid-degrading enzyme or a fragment thereof in step (b) is not removed in step (b) or step (c) or in between step (b) and (c) and the linker which has not connected the solid carrier with the branched-chain amino acid-degrading enzyme or a fragment thereof in step (b), or a part thereof, covalently binds the protective layer to the branched-chain amino acid-degrading enzyme or the fragment thereof in step (c). The amount of the linker which has not connected the solid carrier with the branched-chain amino acid-degrading enzyme or a fragment thereof in step (b) is usually between 30% and 70%, preferably between 40% and 60 %, more preferably around 50% of the amount of linker added to the solid carrier in step (b). In one embodiment there is no washing step between adding the linker to the solid carrier provided in step (a) in (i) and adding the branched-chain amino acid-degrading enzyme or a fragment thereof to the solid carrier and the linker in ii). In one embodiment there is no washing step between any of steps (a) to (c). In one embodiment there is no washing step between adding the linker to the solid carrier provided in step (a) in (i) and adding the branched-chain amino acid-degrading enzyme or a fragment thereof to the solid carrier and the linker in ii) and there is no washing step between any of steps (a) to (c).

In one embodiment the linker is is selected from the group consisting of glutaraldehyde, disuccinimidyl tartrate, bis[sulfosuccinimidyl]suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, activated sulfhydrils, sulfhydryl-reactive 2-pyridyldithiol, BSOCOES (Bis[2-(succinimidooxycarbonyloxy)ethyl]sulfone), DSP (Dithiobis[succinimidyl]propionate]), DTSSP (3,3 '-Dithiobis[sulfosuccinimidyl]propionate]), DTBP (Dimethyl 3,3 '-dithiobispropionimidate 2 HCl), DST (Disuccinimidyl tartarate), Sulfo-LC-SMPT (4-Sulfosuccinimidyl-6-methyl-a-(2-pyridyldithio)toluamido]hexanoate)), SPDP (N-Succinimidyl 3-(2-pyridyldithio)-propionate), LC-SPDP (Succinimidyl 6-(3-[2-pyridyldithio]-propionamido)hexanoate), SMPT (4-Succinimidyloxycarbonyl-methyl-a-[2-pyridyldithio]toluene), DPDPB (1,4-Di-[3'-(2'-pyridyldithio)-propionamido]butane), DTME (Dithio-bismaleimidoethane), BMDB (1,4 bismaleimidyl-2,3-dihydroxybutane) and is preferably glutaraldehyde.

In a preferred embodiment the linker is selected from the group consisting of glutaraldehyde, disuccinimidyl tartrate, bis[sulfosuccinimidyl]suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, BSOCOES (Bis[2-(succinimidooxycarbonyloxy)ethyl]sulfone), DSP (Dithiobis[succinimidyl]propionate]), DTSSP (3,3 '-Dithiobis[sulfosuccinimidyl]propionate]), DTBP (Dimethyl 3,3 '-dithiobispropionimidate 2 HCl), DST (Disuccinimidyl tartarate), BMDB (1,4 bismaleimidyl-2,3-dihydroxybutane) and is preferably glutaraldehyde.

The formation of the protective layer according to step (c) of the present method is usually carried out by forming the respective protective layer with building blocks, wherein the building blocks build the protective layer in a polycondensation reaction as described supa. The immobilization of a functional constituent on the surface of the protective layer according to step (d) of the present method is usually carried out as described supra.

In one embodiment the protective layer is formed by building blocks, wherein as building blocks structural building blocks and protective building blocks are used to form the protective layer, wherein the structural building blocks are precursors of inorganic silica, capable of forming 4 covalent bonds in the layer formed and the protective building blocks are organosilanes as described supra.

In one embodiment the protective layer embeds from about 30% to about 100% of the branched-chain amino acid-degrading enzyme.

In one embodiment the solid carrier is selected from the group of organic particles, inorganic particles, organic-inorganic particles, self-assembled organic particles, silica particles, gold particles, magnetic particles and titanium particles and is preferably a silica particle, more preferably a silica nanoparticle (SNP).

A preferred method of the present invention is a method of producing a composition, the composition comprising a solid carrier, a branched-chain amino acid-degrading enzyme or a fragment thereof immobilized on the surface of the solid carrier, a protective layer to protect the branched-chain amino acid-degrading enzyme or the fragment thereof by embedding the branched-chain amino acid-degrading enzyme or the fragment thereof, and a functional constituent immobilized on the surface of the protective layer, wherein the functional constituent immobilized on the surface of the protective layer is a polymer comprising repeat units, wherein each repeat unit comprises at least one amino group and/or at least one thiol group, the method comprising the following steps:
(a) providing a solid carrier, wherein the solid carrier is provided in suspension, preferably wherein the solid carrier is provided in suspension in water and/or non-ionic surfactants, more preferably wherein the solid carrier is provided in suspension in mixtures of water and non-ionic surfactants;
(b) immobilizing a branched-chain amino acid-degrading enzyme or a fragment thereof on the solid carrier, wherein preferably the surface of the solid carrier is at least partly modified before the branched-chain amino acid-degrading enzyme or a fragment thereof is immobilized on the solid carrier, wherein i) a linker is added to the suspension of the solid carrier or i) a linker is added to the suspension of the solid carrier after the at least partly modification of the surface of the solid carrier and ii) the branched-chain amino acid-degrading enzyme or a fragment thereof, preferably a solution of the branched-chain amino acid-degrading enzyme or a fragment thereof is added to the suspension of the solid carrier and the linker, wherein the linker connects the solid carrier with the branched-chain amino acid-degrading enzyme or a fragment thereof;
(c) forming a protective layer on the surface of the solid carrier to protect the branched-chain amino acid-degrading enzyme or the fragment thereof immobilized on the solid carrier, wherein the linker which has not connected the solid carrier with the branched-chain amino acid-degrading enzyme or a fragment thereof in step (b), or a part thereof, covalently binds the protective layer to the branched-chain amino acid-degrading enzyme or the fragment thereof;
(d) immobilizing a functional constituent on the surface of the protective layer, wherein the functional constituent immobilized on the surface of the protective layer is a polymer comprising repeat units, wherein each repeat unit comprises at least one amino group and/or at least one thiol group.

Also provided is a composition comprising a solid carrier, a branched-chain amino acid-degrading enzyme or a fragment thereof immobilized on the surface of the solid carrier, a protective layer to protect the branched-chain amino acid-degrading enzyme or the fragment thereof by embedding the branched-chain amino acid-degrading enzyme or the fragment thereof, and a functional constituent immobilized on the surface of the protective layer, wherein the functional constituent immobilized on the surface of the protective layer is a polymer comprising repeat units, wherein each repeat unit comprises at least one amino group and/or at least one thiol group, wherein the composition is obtainable by the methods, in particular by the preferred method of the invention as described supra.

### Examples

### Material and Methods:

### Reagents

- Tetraethyl orthosilicate 99% (TEOS), (3-aminopropyl)-triethoxysilane (APTES), ammonium hydroxide (ACS grade, 28-30%), ethanol (ACS grade, anhydrous), glutaraldehyde (grade I, 25% in water), polysorbate 80, acetic acid, leucine, isopentylamine, methanol, formic acid, trichloroacetic acid, dexamethasone were purchased from Sigma-Aldrich.
- Chitosan 95/500 P was purchased from Heppe Medical Chitosan GmbH
- Leucine decarboxylase was produced by RD Biotech from the mouse gene model 853 (Uniprot Q3UNZ2: Ldc1 - Gene model 853, (NCBI) - Mus musculus (Mouse) | UniProtKB ) and supplied in 20 mM Tris/HCl pH 8.0, 150 mM NaCl, DTT 2 mM
- Slide-A-Lyzer^{™} Dialysis Cassettes, 10K MWCO were purchased from ThermoFisher Scientific
- Caco-2 (human colorectal adenocarcinoma cell line) and HT29-MTX-E12 (human colon cancer cell line) were purchased from the American Type Culture Collection (ATCC).
- NoSpin^{™} HepaRG^{™} were purchased from Lonza.
- ThinCert^{™} cell culture insert plates (3.0 µm pore size in PET) were purchased from Greiner bio-one.
- BRANDplates^{®} inserts (0.4 µm pore size in PET) were purchased from Brand^{®}.
- MicroTissues 3D Petri Dish system was purchased from MicroTissues Inc.
- UltraPure Agarose was purchased from Invitrogen.
- Fetal Bovine Serum, Penicillin/Streptomycin (10'000 U/ml Penicillin/10'000 µg/ml Streptomycin), L-Glutamine 200mM (100x), Dulbecco's Phosphate Buffered Saline DPBS (1X), 0.25% Trypsin-EDTA (1X), Dulbecco's Modified Eagle's Medium (DMEM), William's E medium without phenol red + GlutaMAX, were purchased from Gibco.
- Insulin-Transferrin-Sodium Selenite Supplement (ITS) was purchased from Roche.
- CellTiter-Glo^{®} Luminescent Cell Viability Assay was purchased from Promega.
- Human Albumin ELISA kit components were provided by Lubio Science.
- Ketoconazole was provided by Supelco.

### Generation of Human Liver Microtissues (LMTs)

LMTs were generated using fully differentiated, non-proliferative NoSpin^{™} HepaRG^{™} cells, closely resembling primary human hepatocytes. The 3D microtissues were formed using MicroTissues 3D Petri Dish system and UltraPure Agarose. To prepare agarose micro-molds, sterile agarose powder was dissolved in a 0.9% NaCl solution (2% agarose final concentration). Each micro-mold produced 35 microtissue wells, consisting of 3,000 cells per each (total of 1.05 × 10^5 cells per mold).

Two media types were utilized for LMTs generation: a maintenance medium (William's E Medium without phenol red + GlutaMAX, 1X Insulin-Transferrin-Sodium Selenite Supplement (ITS), 100 nM Dexamethasone, and 1% penicillin-streptomycin (P/S) and an aggregation medium (maintenance medium supplemented with 20% FBS). Cells were seeded into the micro-molds in an aggregation medium and left to aggregate in the incubator for six days, with the medium changed three times per week. After 6 days, LMTs were maintained in maintenance medium. At the experimental endpoint, LMTs were removed from the molds by rinsing with 1X PBS containing Mg²⁺ and Ca²⁺ and collected into low-binding Eppendorf tubes for further analysis.

### Generation of the Human Gastro-Intestinal Barrier Model (GI)

The GI model was developed using two cell lines, Caco-2 (human colorectal adenocarcinoma cell line) and HT29-MTX-E12 (human colon cancer cell line), both sourced from ATCC. Cells were cultured in a complete medium consisting of Dulbecco's Modified Eagle's Medium (DMEM) supplemented with 10% heat-inactivated fetal calf serum, 2mM L-glutamine, 1% non-essential amino-acid and 100 U/mL penicillin/streptomycin. The GI model was established in polyethylene terephthalate (PET) transwell inserts with a pore size of 3 µm, suitable for a 24-well plate. Each transwell insert had a growth area of 0.33 cm², with a seeding density of 2.6 × 10⁶ cells per cm², resulting in 8.74 × 10⁵ cells per insert.

Based on prior optimization, a cell ratio of 75% Caco-2 and 25% HT29-MTX-E12 was used, with the specific number of cells per line calculated proportionally. A 250 µL suspension containing cells in a complete medium was pipetted onto the apical side of the insert, while 1500 µL of complete medium was added to the basal side. The medium was refreshed three times weekly. The intestinal barrier model reached maturity for experimental treatment after 21 days of differentiation.

### Treatment of the GI model and LMTs with Leucine and Isopentylamine

To assess the effects of leucine and isopentylamine on cell viability and functionality of LMTs and GI inserts, both models were exposed to concentrations of [2, 5, 10, 20, 30] mM of each compound. For LMTs the treatment solutions were prepared in a maintenance medium and applied for 24 hours after the aggregation period. For GI models the treatment solutions were prepared in DMEM complete medium and applied for 24 hours after 21 days of differentiation.

### Viability Assay: Intracellular ATP Content

The cytotoxicity of leucine and isopentylamine treatments was assessed via an ATP assay, which measures cell viability by quantifying intracellular ATP levels. After treatment:
- LMTs were collected in low-binding Eppendorf tubes, lysed in CellTiter Glow Luminescent reagent and incubated for 15 minutes at RT in an orbital shaker. After 15 minutes, sterile water was added in solutions for 10 additional minutes.
- Transwell membranes containing the GI cell lines were washed 3 times in PBS 1X, incubated with a 1:1 dilution of Cell Titer Glow and nuclease-free water for 15 minutes at RT on an orbital shaker.

In both models, luminescence was measured in a white opaque 96-well plate using a Synergy H1 Multimode Microplate Reader (Bio-Tek Instruments) and viability was calculated relative to untreated controls using Excel. Ketoconazole (250 µM or 500 µM) served as a negative control.

### Albumin release: ELISA

Human albumin levels in the LMT supernatant were quantified using a Human Albumin ELISA kit provided by Lubio Science. Following buffer preparation guidelines, the high-binding plate was coated overnight at 4°C, blocked at RT, and incubated with samples for 1 hour. Detection with HRP antibody (diluted 1:100,000) was performed for 1 hour at RT. TMB substrate was applied in the dark, and the reaction was stopped with 0.18 M H₂SO₄ before reading absorbance at 450 nm using a Synergy H1 Multimode Microplate Reader (Bio-Tek Instruments).

### Integrity of the Human Intestinal Barrier Model: Trans-Epithelial Electrical Resistance (TEER)

TEER was measured after 21 days of differentiation using the CellZscope system (NanoAnalytics). After an initial equilibration period of 2-4 hours, some inserts were treated with leucine, isopentylamine, or ketoconazole (500 µM as a negative control). Data analysis was performed in Excel, with values normalized to the untreated sample at 0 hours. Graphs were generated in GraphPad with time 0 marking the treatment start.

### Synthesis of silica nanoparticles (SNPs)

Silica nanoparticles (50 nm) have been synthetized following the original Stöber process as described in WO2015/014888 A1. Briefly, ethanol, distilled water (6 M) and ammonium hydroxide (0.13 M) were mixed and stirred at 400 rpm for 1 h. TEOS (0.28 M) was added, and the solution was stirred at 400rpm at 20°C for 22h. The solution was then centrifuged at 20000 g for 20 min and washed successively with ethanol and water. Particle size measurement was carried out on SEM micrographs acquired at a magnification of 150000x using the image analysis software Olympus stream motion.

### Production of NP-1

Prior to the reaction, leucine decarboxylase was dialyzed in a dialysis cassette of 10KDa MWCO.

To SNPs (10 mg/mL, 63 nm) in H₂O/PS80 (8 mg/L) was added APTES (3.4 mM). The reaction mixture was allowed to react for 10 min at 20°C, 400 rpm. Then, glutaraldehyde (3.4 mM) was added, and the reaction mixture was stirred for 10 min at 20°C, 400 rpm. A priming was performed by adding APTES (3.4 mM) and stirring the reaction mixture for 10 min at 20°C, 400 rpm. Leucine decarboxylase (3.3 mg/mL, 0.15 mM) was added, and the reaction mixture was allowed to react for 10 min at 20°C, 400 rpm. An organosilica layer was grown at the surface of the immobilized LD using APTES (7.5 mM) and TEOS (79 mM). The resulting suspension was allowed to react for 5 hours at 20°C, 400 rpm. The particles were washed 3 times (by centrifugation during 5 min at 20000 rcf) in H₂O/PS80 (8 mg/L) and resuspended in H₂O/PS80 (8 mg/L). A solution of chitosan in acetic acid (0.1 M) was added to the particle suspension to achieve a final chitosan concentration of 81 µg/mL. The reaction mixture was allowed to react for 30 min at 20°C, 400 rpm. The particles were centrifuged for 5 min at 20000 rcf and washed 3 times in H₂O/PS80 (8 mg/L). NP-1 were cured overnight in a water bath at 20°C.

### Evaluation of the immobilization rate of leucine decarboxylase: Lowry assay

Immobilization yield of enzyme was quantified using the indirect Lowry protein quantification method. A standard regression curve with known concentrations of protein was build using Bovine Serum Albumin (BSA) standards. The supernatant of nanoparticles after enzyme immobilization was taken and centrifuged for 5 minutes at 20k rcf. Then, 1 mL of Lowry solution was added to 200 µL of samples and standards, vortexed and incubated at RT for 15 minutes. Subsequently, 100 µL of Folin reagent 1N were added while vortexing and incubated for 30 minutes at RT. Finally, the absorbance was read at 750 nm using Biotek Synergy H1 Reader.

### Size distribution and thickness layer of NP-1: Scanning Electron Microscope (SEM)

A small aliquot of NP-1 solution in H₂O/PS80 was centrifuged at 20 000g for 5 min and washed three times with sterile water. On each step, the solution was sonicated for 5 minutes in a sonicator bath. Particle size measurement was carried out on SEM micrographs acquired at a magnification of 150 000x using the image analysis software Olympus Stream Motion. The ferret diameter of NP-1 was evaluated using the software Image J and further calculations for the size distribution histogram were performed in Excel.

### Leucine decarboxylase enzyme activity assay

A substrate solution (50 µL) containing 4 µM pyridoxal-5'-phosphate and 100 mM leucine in 20 mM phosphate buffer (pH 7) was added to the enzyme solution (50 µL). The reaction mixture was incubated for 30 min at 37 °C, 750 rpm. Samples (15 µL) were withdrawn at 0, 5, 10, 15, 20, and 30 minutes. To quench the reaction, 15 µL of 15% trichloroacetic acid (TCA) was added to each sample. The samples were then centrifuged at 20000 rcf for 5 minutes, and 20 µL of the supernatant was collected for analysis.

For *in vitro* applications samples were collected from the basal compartment of the integrated model in a Eppendorf containing a 15% solution of trichloroacetic acid (TCA) to quench the enzymatic reaction. Samples (15 µL) were withdrawn at 0, 5, 10, 15, 20, and 30 minutes. After each sample's collection, fresh maintenance medium without phenol red was added back on the basal side. The collected samples were then centrifuged at 20000 rcf for 5 minutes, and 20 µL of the supernatant was collected for analysis.

The samples were analyzed using an Agilent 1260 Infinity II Prime UHPLC system equipped with an Eclipse Plus C18 RRHD column (2.1 × 50 mm, 1.8 µm particle size) to determine isopentylamine concentration. The mobile phase consisted of water containing 0.1% formic acid (solvent A) and methanol (solvent B). The analysis was performed using a gradient elution:
- 0-1 min: 5% B
- 1-1.1 min: 5-90% B
- 1.1-2.1 min: 90% B
- 2.1-4.1 min: 90-5% B

The flow rate was 0.7 mL/min, the column temperature was 40 °C, and the injection volume was 1 µL.

### Human integrated model composed of GI inserts and LMTs

For the development of the integrated *in vitro* model, the procedure for generating the gastrointestinal (GI) inserts followed the above-described methodology. However, to accommodate the placement of the inserts above the mold containing 35 microtissues in a 24-well plate, a different type of Transwell insert was utilized. Specifically, PET Transwell inserts with a pore size of 0.4 µm and a growth area of 0.6 cm² (BRANDplates) were selected due to their reduced height compared to the previously used inserts (ThinCert^{™}). Importantly, the seeding density remained unchanged, with 2.6 × 10⁶ cells per cm². Following 21 days of differentiation for the GI inserts and 6 days of aggregation for LMTs, the integrated model was established by layering transwell inserts over the LMTs. Treatments on the apical side included 0.5 mg/mL NP-1 (6.4 mU), 5 mM leucine or isopentylamine, and 4 mM Pyridoxal Phosphate (PLP), all prepared in DMEM complete medium without phenol red. The basal side, composed of maintenance medium, was used as a collecting sample point.

Sample collection involved adding 50 µL of treatment medium from the basal side to 50 µL of 15% trichloroacetic acid (TCA) in an Eppendorf tube to quench the enzymatic reaction. Sampling was performed at multiple time points (10 min; 30 min; 1h; 2h; 3h; 4h; 6h, and 24h), with 50 µL of fresh medium added after each collection. Samples were centrifuged at 4°C for 5 minutes at 20,000 rcf, and 80 µL of supernatant was analyzed by LC-MS for isopentylamine evaluation.

After 24 hours of treatment, medium from the basal side was collected for albumin ELISA, and ATP assays were performed on both the GI inserts and LMTs, using previously described protocols.

### Results

### Example 1: Safety of Leucine and Isopentylamine in single organ in vitro models

Single-organ *in vitro* models, GI and LMTs, were employed to assess potential toxic effects of the substrate leucine and its reaction product, isopentylamine, catalyzed by NP-1. This toxicological assessment was essential for establishing safe concentration ranges for subsequent experiments involving the integrated *in vitro* model. Given that the average of the daily leucine intake for a typical 70 kg European individual is approximately 5 mM/day, concentrations around this value were selected for testing. Concentrations above 5 mM were considered potentially harmful, as they exceed physiological limits. The same concentration range was used for isopentylamine, assuming full conversion of leucine to isopentylamine by NP-1.

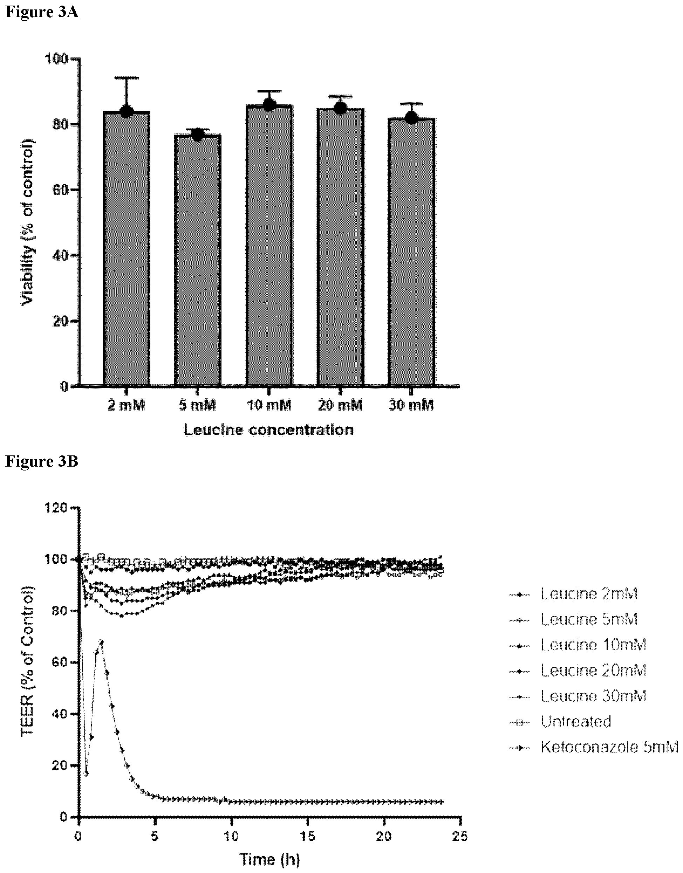

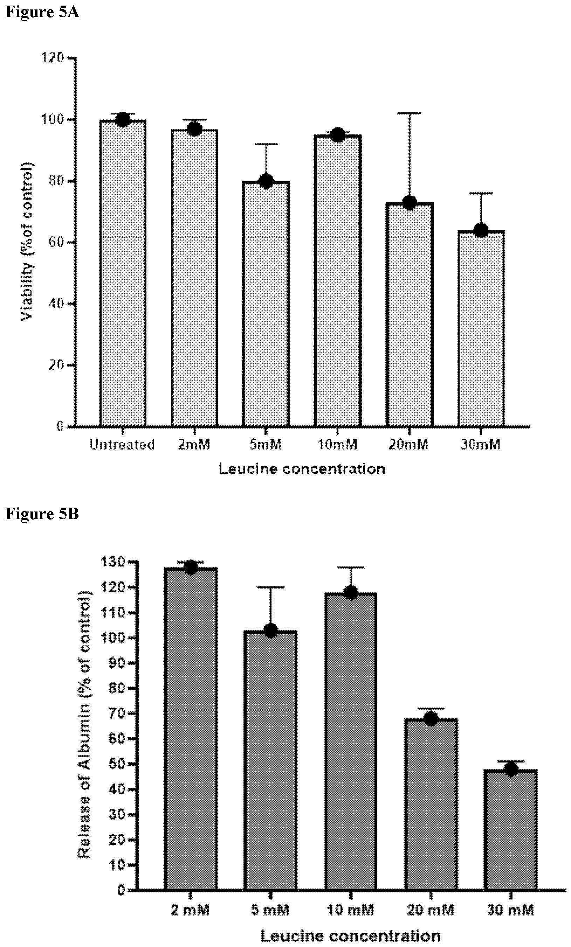

The intracellular level of ATP, proportional to the number of cell viable, demonstrated the leucine treatment safety: none of the concentrations tested (up to 30 mM) was recorded being toxic to the GI model (Figure 3A), while on LMTs, toxicity levels were registered only for concentrations higher than 20 mM (Figure 5A). Barrier integrity, measured by TEER, further indicated that the tight junctions of the GI model remained intact across all tested concentrations (Figure 3B).

Similarly to the intracellular levels of ATP, in LMTs, a dose-dependent effect was observed on albumin release: concentrations above 20 mM reduced albumin production and release, compromising liver function, which is expected at non-physiological levels (Figure 5B).

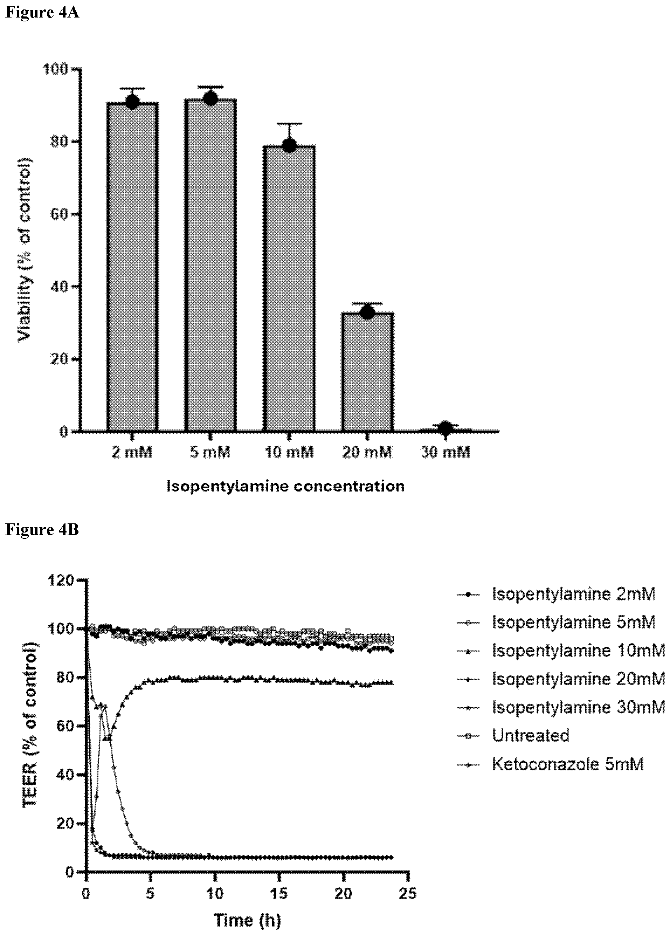

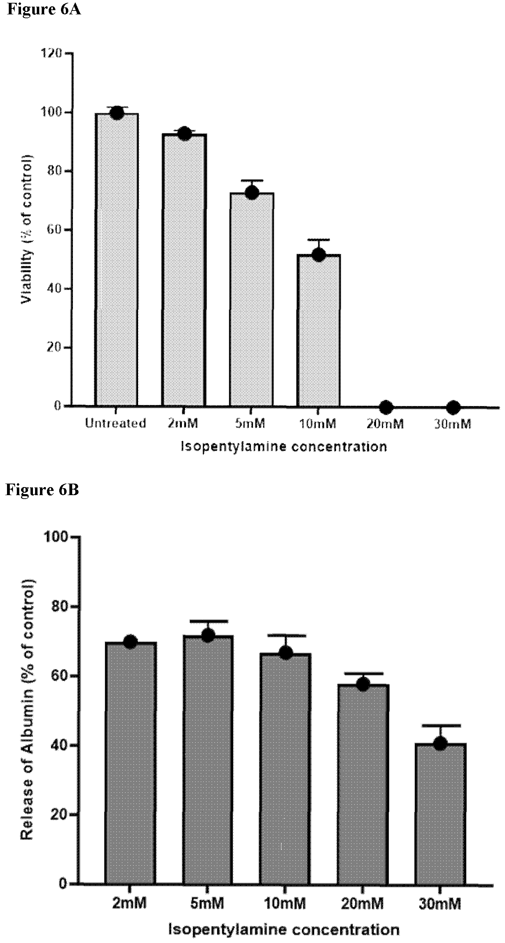

Isopentylamine, the metabolic product of leucine, exhibited dose-dependent toxicity in both single-organ models. In the GI model, ATP assays indicated toxicity at concentrations exceeding 10 mM (Figure 4A), although a decrease in barrier integrity, as measured by TEER, was observed only at concentrations above 20 mM. Similarly, in the LMTs, toxicity was detected at concentrations higher than 5 mM (Figure 6 A), while a reduction in albumin production and release became apparent since concentrations of 2 mM.

These preliminary findings established the safe concentration range for leucine and isopentylamine and demonstrated that both *in vitro* models mimic physiological responses to varying leucine and isopentylamine concentrations. The observation of toxicity at supraphysiological levels highlights its reliability in replicating the expected physiological response to leucine levels that exceed normal intake. However, it should be noted that the static nature of these models results in prolonged compound exposure to cellular surfaces compared to physiological conditions, which may accentuate the observed toxicity responses of isopentylamine.

### Example 2: Production and characterization of NP-1

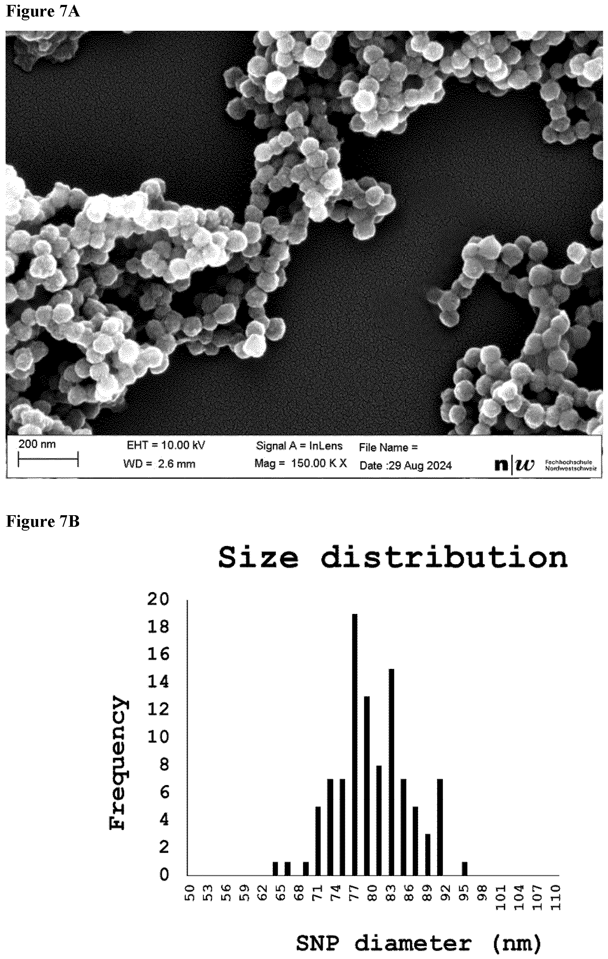

NP-1 was successfully produced following the protocol described above, achieving a concentration of 15.5 mg/mL. Initial scanning electron microscopy (SEM) imaging revealed a distinct spherical morphology of the SNPs (Figure 7A). From these images, an average particle diameter of 81.5 nm was calculated, with a monodisperse size distribution (Figure 7B). The Lowry assay, which estimates protein content in solution, demonstrated a leucine decarboxylase immobilization efficiency of 93.4% on NP-1, relative to the total amount used for immobilization. Further analysis by LC/MS confirmed the efficacy of NP-1, indicating an enzymatic activity of 51 U/g of SNP, meaning 1 g of SNPs produces 51 µmol of isopentylamine per minute.

### Example 3: Safety of NP-1 on the integrated in vitro GI and LMTs model

The integrated human *in vitro* model consists of gastrointestinal (GI) inserts positioned above liver microtissues (LMTs), simulating the interaction between intestinal absorption and hepatic metabolism. Treatment safety for NP-1 was evaluated using a non-toxic concentration of leucine at 5 mM, as established from prior data presented in Example 1. In this model, the apical side was treated with 5 mM leucine (the substrate), 0.5 mg/mL NP-1 (equivalent to 6.4 mU), and 4 mM pyridoxal phosphate (PLP), a cofactor naturally present in the GI tract. The metabolic product, isopentylamine, was measured directly on the basal side, where the LMTs are situated. To better understand inter-organ signaling within the integrated model, additional controls were applied in the form of single-component or single-organ treatments.

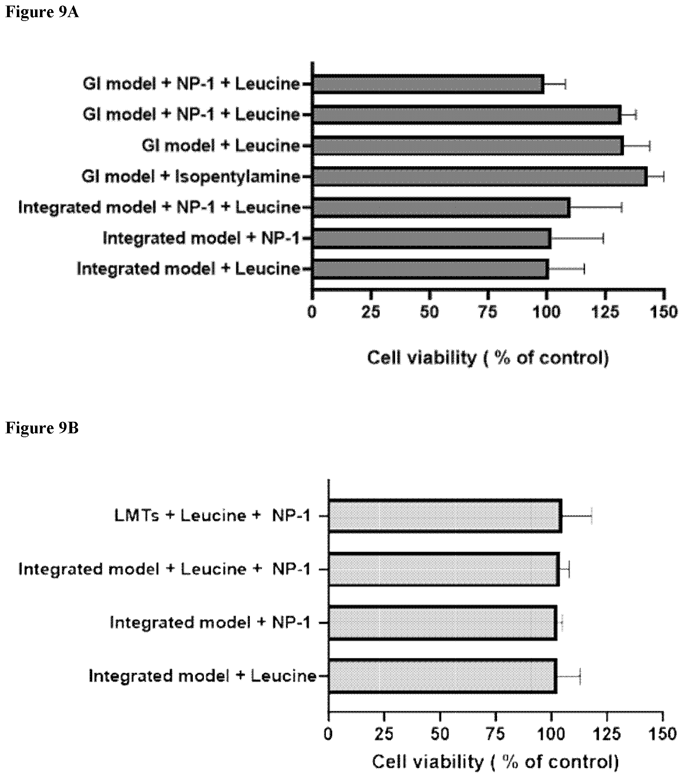

As illustrated in Figures 9A and 9B, intracellular ATP levels confirmed the safety of leucine and isopentylamine treatment, aligning with previously obtained results in Example 1. Furthermore, the data indicate that NP-1 is safe when applied either in single-component or integrated models, under both isolated and combined administrations.

### Example 4: Efficacy of NP-1 in an integrated model of LMTs and GI inserts

Assessing the permeability of a drug product across the intestinal barrier is essential for predicting its transport mechanisms and enhancing its therapeutic efficacy. Some molecules traverse the intestinal barrier through passive diffusion, while others depend on active transport mechanisms.

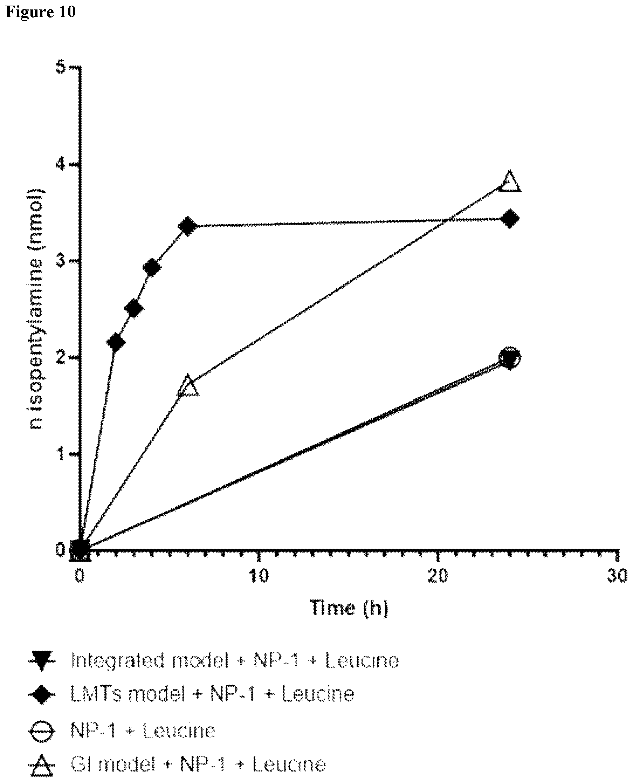

The goal of NP-1 nanomedicine is to metabolize leucine directly in the intestine, preventing systemic absorption and accumulation in the body. Beyond demonstrating nanoparticle efficacy, quantifying isopentylamine the metabolite that crosses the intestinal barrier-provides insight into potential interactions between this exogenous compound and other organs. Measurements of isopentylamine on the basal side serve as indicators of NP-1 efficacy, reflecting both production and permeability across the intestinal barrier in the integrated model. These results are shown in Figure 10. 5 mM of leucine (in 250 µL) was used as the substrate. This concentration corresponds to the daily average leucine uptake for a 70 kg European individual, as described in Example 1. Quantifiable LC-MS data above the detection limit were observed in all conditions containing both NP-1 and leucine substrate.

In a control model with an empty Transwell insert (NP-1 + Leucine), which utilizes only the PET membrane as a physical barrier, leucine metabolism by NP-1 demonstrated passive diffusion of isopentylamine through membrane pores. In the single GI model under similar conditions (GI model + NP-1 + Leucine), a higher concentration of isopentylamine accumulated on the basal side, continuing to increase over time. This increase may be attributed not only to passive transport, but also to active transport promoted by GI model cells.

When the same conditions were applied to the single LMT model (LMTs + NP-1 + Leucine), basal isopentylamine levels rose rapidly within the first four hours, reaching a plateau after six hours. This plateau likely reflects equilibrium between apical and basal sides and/or LMT-mediated isopentylamine absorption or further metabolism.

In the integrated model (integrated model + NP-1 + Leucine), the response to treatment differed from that of the single-organ models, likely representing a balance between GI cell active transport and LMT absorption. However, this more complex inter-organ interaction with isopentylamine warrants further investigation.

All conditions treated with NP-1 demonstrated both safety (Example 3) and efficacy in metabolizing a physiologically relevant amount of leucine (5 mM) and facilitating the absorption of its metabolic product, isopentylamine.

## Claims

1. A composition comprising a solid carrier, a branched-chain amino acid-degrading enzyme or a fragment thereof immobilized on the surface of the solid carrier, a protective layer to protect the branched-chain amino acid-degrading enzyme or the fragment thereof by embedding the branched-chain amino acid-degrading enzyme or the fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group.

2. The composition according to claim 1, wherein the polymer comprising repeat units wherein each repeat unit comprises at least one amino group and/or at least one thiol group is a polyglucosamin selected from the group consisting of chitin, chitosan, polyglucosaminoglycans, chondroitin, heparin, keratan and dermatan or a derivative thereof.

3. The composition according to anyone of claims 1-2, wherein the polymer comprising repeat units wherein each repeat unit comprises at least one amino group and/or at least one thiol group is chitosan or a derivative thereof.

4. The composition according to anyone of claims 1-3, wherein the branched-chain amino acid-degrading enzyme or a fragment thereof is a branched-chain amino acid decarboxylase.

5. The composition according to anyone of claims 1-3, wherein the branched-chain amino acid-degrading enzyme or a fragment thereof is selected from the goup consisting of leucine decarboxylase or a fragment thereof and valine decarboxylase or a fragment thereof.

6. The composition according to anyone of claims 1-3, wherein the branched-chain amino acid-degrading enzyme or a fragment thereof is leucine decarboxylase or a fragment thereof

7. The composition of anyone of claims 1-6, for use as a medicament.

8. The composition of anyone of claims 1-6, for use in a method of treatment of branched-chain amino acid metabolism related diseases.

9. The composition of anyone of claims 1-6, for use in a method of treatment of Maple Syrup Urine Disease (MSUD).

10. A method of producing a composition, the composition comprising a solid carrier, a branched-chain amino acid-degrading enzyme or a fragment thereof immobilized on the surface of the solid carrier, a protective layer to protect the branched-chain amino acid-degrading enzyme or the fragment thereof by embedding the branched-chain amino acid-degrading enzyme or the fragment thereof, and a functional constituent immobilized on the surface of the protective layer, wherein the functional constituent immobilized on the surface of the protective layer is a polymer comprising repeat units, wherein each repeat unit comprises at least one amino group and/or at least one thiol group, the method comprising the following steps:
(a) providing a solid carrier;
(b) immobilizing a branched-chain amino acid-degrading enzyme or a fragment thereof on the solid carrier;
(c) forming a protective layer on the surface of the solid carrier to protect the branched-chain amino acid-degrading enzyme or the fragment thereof immobilized on the solid carrier;
(d) immobilizing a functional constituent on the surface of the protective layer, wherein the functional constituent immobilized on the surface of the protective layer is a polymer comprising repeat units, wherein each repeat unit comprises at least one amino group and/or at least one thiol group.

11. The method of claim 10, wherein there is no washing step between adding the linker to the solid carrier provided in step (a) in (i) and adding the branched-chain amino acid-degrading enzyme or a fragment thereof to the solid carrier and the linker in ii).

12. The method of anyone of claims 10-11, wherein there is no washing step between any of steps (a) to (c).

13. A composition comprising a solid carrier, a branched-chain amino acid-degrading enzyme or a fragment thereof immobilized on the surface of the solid carrier, a protective layer to protect the branched-chain amino acid-degrading enzyme or the fragment thereof by embedding the branched-chain amino acid-degrading enzyme or the fragment thereof, and a functional constituent immobilized on the surface of the protective layer, wherein the functional constituent immobilized on the surface of the protective layer is a polymer comprising repeat units, wherein each repeat unit comprises at least one amino group and/or at least one thiol group, wherein the composition is obtainable by the method of any one of claims 10-12.
